# EUROPÄISCHE PATENTSCHRIFT

(11) **EP 2 890 700 B1**
(45) Veröffentlichungstag und Bekanntmachung des Hinweises auf die Patenterteilung: **13.12.2017**
(21) Anmeldenummer: 12766007.4
(22) Anmeldetag: 31.08.2012
(51) Int. Cl.: C07D 498/14, A61K 31/5383, A61P 25/28

(54) **HALOGENIERTE BENZOXAZINE UND IHRE VERWENDUNG**
HALOGENATED BENZOXAZINES AND THEIR USE
DÉRIVÉS HALOGÉNÉS DE BENZOXAZINE ET LEUR UTILISATION

(30) Priorität: 28.08.2012 WO PCT/EP2012/066713
(43) Veröffentlichungstag der Anmeldung: 08.07.2015
(73) Patentinhaber: Eberhard Karls Universität Tübingen Medizinische Fakultät, 72074 Tübingen (DE)
(72) Erfinder: KESENHEIMER, Christian, 72074 Tübingen-Pfrondorf (DE); MAIER, Florian, 72766 Reutlingen-Oststadt (DE); STUMM, Ramona, 72820 Sonnenbuehl (DE); PICHLER, Bernd, 85298 Scheyern (DE)
(74) Vertreter: Witte, Weller & Partner Patentanwälte mbB
(86) Internationale Anmeldenummer: PCT/EP2012/067006
(87) Internationale Veröffentlichungsnummer: WO 2014/032732

(56) Entgegenhaltungen:
- EP-A1- 1 193 260
- WO-A1-2005/016934
- WO-A2-02/16333
- HINTERSTEINER, MARTIN ET AL: "In vivo detection of amyloid-.beta. deposits by near-infrared imaging using an oxazine-derivative probe", NATURE BIOTECHNOLOGY , 23(5), 577-583 CODEN: NABIF9; ISSN: 1087-0156, 2005, XP002695064,

## Beschreibung

Die vorliegende Erfindung betrifft neue halogenierte Benzoxazin-Tracer Verfahren zu ihrer Herstellung, ihre Verwendung zur Diagnose von Krankheiten sowie ihre Verwendung zur Herstellung von Arzneimitteln zur Diagnose von Krankheiten, vorzugsweise Demenzerkrankungen und insbesondere der Alzheimer-Krankheit, bei Menschen und/oder Tieren.

In einer alternden Gesellschaft kommt der Diagnose und Therapie von Erkrankungen, die üblicherweise erst bei älteren Menschen auftreten, eine immer größere Bedeutung zu. Zu diesen Erkrankungen gehören unter anderem die neurodegenerativen Erkrankungen und insbesondere die Alzheimer-Krankheit, die etwa 70% aller neurodegenerativen Erkrankungen ausmacht.

Die Alzheimer-Krankheit ist eine durch eine zunehmende Verschlechterung der kognitiven Leistungsfähigkeit, Demenz und Verlust der Gedächtnisfähigkeiten gekennzeichnete neurodegenerative Erkrankung. Auf zellulärem Level ist die Alzheimer-Krankheit durch die Bildung von Plaques aus Beta-Amyloid-Peptiden (Aß-Plaques) gekennzeichnet, die sich bereits viele Jahre vor Auftreten der klinischen Symptome bilden können.

Ein Problem in der Diagnose und *in extenso* der Therapie der Alzheimer-Krankheit, sowie in der Entwicklung wirksamer Therapien für die Alzheimer-Krankheit liegt darin, dass bis heute eine abschließende Diagnose nur postmortem im Rahmen einer Autopsie durch Anfärben der Aß-Plaques möglich ist. Die Möglichkeit Aß-Plaques im lebenden Organismus nachzuweisen wäre insbesondere daher von Interesse, da die Bildung der Aß-Plaques deutlich vor dem Ausbruch der klinischen Symptome beginnt und somit durch einen solchen Nachweis eine deutlich frühere Erkennung und Therapie der Alzheimer-Krankheit möglich wäre.

Es gibt bereits verschiedene Ansätze um Aß-Plaques zu markieren und sichtbar zu machen. So sind in der WO 2005/016934 und in HINTERSTEINER, MARTIN ET AL: "In vivo detection of amyloid-.beta. deposits by near-infrared imaging using an oxazine-derivative probe",NATURE BIOTECHNOLOGY , 23(5), 577-583 CODEN: NABIF9; ISSN: 1087-0156, 2005 Verbindungen beschrieben, die strukturell den vorliegenden Verbindungen ähnlich sind und die als Fluoreszenzfarbstoffe für Aß-Plaques beschrieben sind. Es hat sich allerdings bei diesen Verbindungen gezeigt, dass diese für die Alzheimer-Diagnostik im Menschen ungeeignet sind, da ihre Fluoreszenz nicht durch den menschlichen Schädel, also im lebenden Menschen, beobachtet werden kann.

Ein weiterer Ansatz in der Forschung zur Alzheimer-Diagnostik besteht in der Entwicklung von markierten Tracer-Verbindungen, insbesondere radioaktiv markierten Tracer-Verbindungen, zur Verwendung mit nicht-invasiven Detektionsverfahren wie bspw. Positronen-Emissions-Tomographie (PET) oder Magnetresonanztomographie (MRT). Die auf diesem Gebiet zurzeit vielversprechendsten Verbindungen sind Thioflavin-Derivate, wobei die am weitesten entwickelte Verbindung ein radiomarkiertes Thioflavin-Derivat ist, das als Pittsburgh Compound B (PIB) bezeichnet wird. Verbindungen dieser Art sind bspw. in der WO 02/16333 und der WO 2004/083195 beschrieben. Diese Verbindungen haben allerdings den Nachteil, dass diese ab einer gewissen Konzentration an Aß-Plaques einen Sättigungseffekt zeigen, das heißt, dass trotz einer Zunahme der Menge an Aß-Plaques keine Zunahme im Tracer-Signal mehr zu erkennen ist. Des Weiteren können mit diesen Verbindungen an Gefäßen abgelagerte Aß-Plaques nicht detektiert werden. Im Hinblick auf diese Nachteile sind diese Verbindungen zwar zur allgemeinen Diagnose geeignet, es ist allerdings fraglich, ob z.B. der Krankheitsverlauf damit zuverlässig verfolgt werden kann.

Somit besteht ein dringender Bedarf an neuen Verbindungen zum Einsatz in der Alzheimer-Diagnostik, die die oben genannten Nachteile nicht aufweisen und mit denen *in vivo* eine Alzheimer-Erkrankung frühzeitig diagnostiziert und über den gesamten Krankheitsverlauf verfolgt werden kann.

Überraschenderweise wurde gefunden, dass die in der vorliegenden Erfindung beschriebenen halogenierten Benzoxazine als Tracer für Aβ-Plaques z.B. für die PET und MRT geeignet sind. Insbesondere hat sich gezeigt, dass diese Verbindungen über einen breiten Konzentrationsbereich ein zur Konzentration an Aβ-Plaques korreliertes Signal liefern.

Gegenstand der Erfindung sind radioaktiv markierte tracer, aufweisend ein Salz der Verbindung der Formel 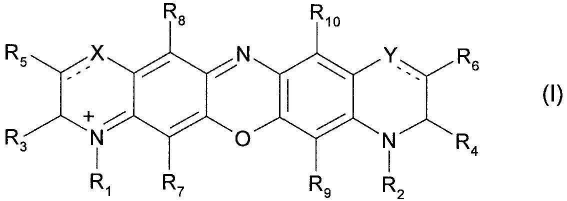 in welcher,
X und Y unabhängig voneinander für CH, CH₂, N, S oder O stehen,
   wobei X und Y nicht gleichzeitig CH oder CH₂ sind,
   wobei, wenn X für CH oder N steht, die gestrichelte Linie zwischen X und dem Nachbaratom eine Bindung darstellt, und
   wobei, wenn Y für CH oder N steht, die gestrichelte Linie zwischen Y und dem Nachbaratom eine Bindung darstellt,
R₁ und R₂ unabhängig voneinander ausgewählt sind aus der Gruppe bestehend aus (C₁-C₂)-Alkyl, (C₁-C₂)-Alkoxy und (C₁-C₂)-Alkylsulfonyl,
   wobei Alkyl, Alkoxy und Alkylsulfonyl mit ein bis drei Substituenten substituiert sein können, die ausgewählt sind aus der Gruppe bestehend aus ⁷⁶Br, ⁷⁵Br, ¹⁹F und ¹⁸F,
R₃, R₄, R₇, R₈, R₉ und R₁₀ unabhängig voneinander ausgewählt sind aus der Gruppe bestehend aus Wasserstoff, Halogen, Hydroxy, Amino, Cyano, Nitro, (C₁-C₄)-Alkyl, (C₁-C₄)-Alkylamino und (C₁-C₄)-Alkoxy,
   wobei Alkyl und Alkoxy mit ein bis drei Substituenten substituiert sein können, die ausgewählt sind aus der Gruppe bestehend aus ⁷⁶Br, ⁷⁵Br, ¹⁹F und ¹⁸F,
R₅, wenn X für CH oder N steht, für einen Substituenten steht, der ausgewählt ist aus der Gruppe bestehend aus Wasserstoff, Halogen, Hydroxy, Amino, Cyano, Nitro, (C₁-C₄)-Alkyl, (C₁-C₄)-Alkylamino und (C₁-C₄)-Alkoxy, wobei Alkyl und Alkoxy mit ein bis drei Substituenten substituiert sein können, die ausgewählt sind aus der Gruppe bestehend aus ⁷⁶Br, ⁷⁵Br, ¹⁹F und ¹⁸F,
R₅, wenn X für CH₂, S oder O steht, für zwei Substituenten steht, die unabhängig voneinander ausgewählt sind aus der Gruppe bestehend aus Wasserstoff, Halogen, Hydroxy, Amino, Cyano, Nitro, (C₁-C₄)-Alkyl, (C₁-C₄)-Alkylamino und (C₁-C₄)-Alkoxy,
   wobei Alkyl und Alkoxy mit ein bis drei Substituenten substituiert sein können, die ausgewählt sind aus der Gruppe bestehend aus ⁷⁶Br, ⁷⁵Br, ¹⁹F und ¹⁸F,
R₆, wenn Y für CH oder N steht, für einen Substituenten steht der ausgewählt ist aus der Gruppe bestehend aus Wasserstoff, Halogen, Hydroxy, Amino, Cyano, Nitro, (C₁-C₄)-Alkyl, (C₁-C₄)-Alkylamino und (C₁-C₄)-Alkoxy,
   wobei Alkyl und Alkoxy mit ein bis drei Substituenten substituiert sein können, die ausgewählt sind aus der Gruppe bestehend aus ⁷⁶Br, ⁷⁵Br, ¹⁹F und ¹⁸F, und
R₆, wenn Y für CH₂, S oder O steht, für zwei Substituenten steht, die unabhängig voneinander ausgewählt sind aus der Gruppe bestehend aus Wasserstoff, Halogen, Hydroxy, Amino, Cyano, Nitro, (C₁-C₄)-Alkyl, (C₁-C₄)-Alkylamino und (C₁-C₄)-Alkoxy
   wobei Alkyl und Alkoxy mit ein bis drei Substituenten substituiert sein können, die ausgewählt sind aus der Gruppe bestehend aus ⁷⁶Br, ⁷⁵Br, ¹⁹F und ¹⁸F, wobei die Verbindungen der Formel (I) zumindest einen Substituenten ausgewählt aus der Gruppe bestehend aus ⁷⁶Br, ⁷⁵Br, ¹⁹F und ¹⁸F aufweisen,
oder Solvate des Salzes.

Die im folgenden beschriebenen Verbindungen sind die Verbindungen der Formel (I) und deren Salze, Solvate und Solvate der Salze, sowie die von Formel (I) umfassten, nachfolgend als Ausführungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Sofern die Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der Verbindungen bevorzugt. Umfasst sind aber auch Salze, die für pharmazeutische Anwendungen selbst nicht geeignet sind, aber beispielsweise für die Isolierung oder Reinigung der Verbindungen verwendet werden können.

Physiologisch unbedenkliche Salze der Verbindungen umfassen vorzugsweise Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Als Solvate werden im Rahmen der Erfindung solche Formen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:

Alkyl sowie die Alkylteile in Alkoxy und Alkylsulfonyl stehen für geradliniges oder verzweigtes Alkyl und umfassen, wenn nicht anders angegeben, (C₁-C₆)-Alkyl, insbesondere (C₁-C₄)-Alkyl, wie z.B. Methyl, Ethyl, n-Propyl, Isopropyl, n-Butyl, Isobutyl, tert.-Butyl.

Alkoxy steht im Rahmen der Erfindung vorzugsweise für einen geradkettigen oder verzweigten Alkoxyrest insbesondere mit 1 bis 6, 1 bis 4 bzw. 1 bis 3 Kohlenstoffatomen. Bevorzugt ist ein geradkettiger oder verzweigter Alkoxyrest mit 1 bis 3 Kohlenstoffatomen. Beispielhaft und vorzugsweise seien genannt: Methoxy, Ethoxy, n-Propoxy, Isopropoxy, t-Butoxy, n-Pentoxy und n-Hexoxy.

Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, tert-Butylamino, n-Pentylamino, n-Hexylamino, *N,N*-Dimethylamino, *N,N*-Diethylamino, *N*-Ethyl-*N-*methylamino, *N*-Methyl-*N*-n-propylamino, *N*-Isopropyl-*N*-n-propylamino, *N-tert*-Butyl-*N-*methylamino, *N*-Ethyl-*N*-n-pentylamino und *N*-n-Hexyl-*N*-methylamino. C₁-C₃-Alkylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent.

Halogen steht für Fluor, Chlor, Brom oder Jod und deren Isotope, wobei Fluor und Brom bevorzugt sind, wenn nichts anderes angegeben ist. Insbesondere kann Halogen im Rahmen der Erfindung für ⁷⁶Br, ⁷⁵Br, ¹⁹F und ¹⁸F stehen.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Die in den jeweiligen Kombinationen bzw. bevorzugten Kombinationen von Resten im Einzelnen angegebenen Restedefinitionen werden unabhängig von den jeweilig angegebenen Kombinationen der Reste beliebig auch durch Restedefinitionen anderer Kombinationen ersetzt.

Gegenstand der Erfindung sind auch die entsprechenden tracer, worin die Verbindungen der Formel (I), in welcher
X und Y unabhängig voneinander für CH, CH₂, N, S oder O stehen,
   wobei X und Y nicht gleichzeitig CH oder CH₂ sind,
   wobei, wenn X für CH oder N steht, die gestrichelte Linie zwischen X und dem Nachbaratom eine Bindung darstellt, und
   wobei, wenn Y für CH oder N steht, die gestrichelte Linie zwischen Y und dem Nachbaratom eine Bindung darstellt
R₁ und R₂ unabhängig voneinander ausgewählt sind aus der Gruppe bestehend aus (C₁-C₂)-Alkyl, (C₁-C₂)-Alkoxy und (C₁-C₂)-Alkylsulfonyl,
   wobei Alkyl, Alkoxy und Alkylsulfonyl mit ein bis drei Substituenten substituiert sein können, die ausgewählt sind aus der Gruppe bestehend aus ⁷⁶Br, ⁷⁵Br, ¹⁹F und ¹⁸F,
R₃, R₄, R₇, R₈, R₉ und R₁₀ unabhängig voneinander ausgewählt sind aus der Gruppe bestehend aus Wasserstoff, Halogen, Hydroxy, Amino, (C₁-C₂)-Alkyl, und (C₁-C₂)-Alkoxy, wobei Alkyl und Alkoxy mit ein bis drei Substituenten substituiert sein können, die ausgewählt sind aus der Gruppe bestehend aus ⁷⁶Br, ⁷⁵Br, ¹⁹F und ¹⁸F,
R₅, wenn X für CH oder N steht, für einen Substituenten steht, der ausgewählt ist aus der Gruppe bestehend aus Wasserstoff, Halogen, Hydroxy, Amino, (C₁-C₂)-Alkyl, und (C₁-C₂)-Alkoxy,
   wobei Alkyl und Alkoxy mit ein bis drei Substituenten substituiert sein können, die ausgewählt sind aus der Gruppe bestehend aus ⁷⁶Br, ⁷⁵Br, ¹⁹F und ¹⁸F,
R₅, wenn X für CH₂, S oder O steht, für zwei Substituenten steht die unabhängig voneinander ausgewählt sind aus der Gruppe bestehend aus Wasserstoff, Halogen, Hydroxy, Amino, (C₁-C₂)-Alkyl, und (C₁-C₂)-Alkoxy,
   wobei Alkyl und Alkoxy mit ein bis drei Substituenten substituiert sein können, die ausgewählt sind aus der Gruppe bestehend aus ⁷⁶Br, ⁷⁵Br, ¹⁹F und ¹⁸F,
R₆, wenn Y für CH oder N steht, für einen Substituenten steht der ausgewählt ist aus der Gruppe bestehend aus Wasserstoff, Halogen, Hydroxy, Amino, (C₁-C₂)-Alkyl, und (C₁-C₂)-Alkoxy,
   wobei Alkyl und Alkoxy mit ein bis drei Substituenten substituiert sein können, die ausgewählt sind aus der Gruppe bestehend aus ⁷⁶Br, ⁷⁵Br, ¹⁹F und ¹⁸F,
R₆, wenn Y für CH₂, S oder O steht, für zwei Substituenten steht die unabhängig voneinander ausgewählt sind aus der Gruppe bestehend aus Wasserstoff, Halogen, Hydroxy, Amino, (C₁-C₂)-Alkyl, und (C₁-C₂)-Alkoxy,
   wobei Alkyl und Alkoxy mit ein bis drei Substituenten substituiert sein können, die ausgewählt sind aus der Gruppe bestehend aus ⁷⁶Br, ⁷⁵Br, ¹⁹F und ¹⁸F,
ausgewählt aus der Gruppe bestehend aus ⁷⁶Br, ⁷⁵Br, ¹⁹F und ¹⁸F aufweisen.

Gegenstand der Erfindung sind auch die tracer worin die Verbindungen der Formel (I), in
welcher
X und Y unabhängig voneinander für CH₂, S oder O stehen, wobei X und Y nicht gleichzeitig CH₂ sind,
R₁ und R₂ unabhängig voneinander ausgewählt sind aus der Gruppe bestehend aus (C₁-C₂)-Alkyl, (C₁-C₂)-Alkoxy und (C₁-C₂)-Alkylsulfonyl, wobei zumindest einer der durch R₁ und R₂ dargestellten Reste mit zumindest einem Substituenten substituiert ist, der ausgewählt ist aus der Gruppe bestehend aus ⁷⁶Br, ⁷⁵Br, ¹⁹F und ¹⁸F, und
R₃, R₄, R₅, R₆, R₇, R₈, R₉ und R₁₀ für Wasserstoff stehen.

Gegenstand der Erfindung sind auch tracer worin die Verbindungen der Formel (I), die ausgewählt sind aus der Gruppe bestehend aus:
8-Ethyl-4-(2-fluoroethyl)-3,8,9,10-tetrahydro-2H-bis([1,4]-oxazino)[2,3-*b*:3',2'-*i*]phenoxazin-4-ium,
4-(2-Fluoroethyl)-8-(2-hydroxyethyl)-3,8,9,10-tetrahydro-2H-bis([1,4]oxazino)[2,3-*b*:3',2'-*i*]phenoxazin-4-ium,
[F-18]-8-Ethyl-4-(2-fluoroethyl)-3,8,9,10-tetrahydro-2H-bis([1,4]-oxazino)[2,3-*b*:3',2'-*i*]phenoxazin-4-ium,
[F-18]-4-(2-fluoroethyl)-8-(2-hydroxyethyl)-3,8,9,10-tetrahydro-2H-bis([1,4]oxazino)[2,3-*b*:3',2'-*i*]phenoxazin-4-ium,
[Br-75]-8-Ethyl-4-(2-bromoethyl)-3,8,9,10-tetrahydro-2H-bis([1,4]-oxazino)[2,3-*b*:3',2'-*i*]phenoxazin-4-ium,
[Br-75]-4-(2-Bromoethyl)-8-(2-hydroxyethyl)-3,8,9,10-tetrahydro-2H-bis([1,4]oxazino)[2,3-*b*:3',2'-*i*]phenoxazin-4-ium,
[Br-76]-8-Ethyl-4-(2-bromoethyl)-3,8,9,10-tetrahydro-2H-bis([1,4]-oxazino)[2,3-*b*:3',2'-*i*]phenoxazin-4-ium, und
[Br-76]-4-(2-bromoethyl)-8-(2-hydroxyethyl)-3,8,9,10-tetrahydro-2H-bis([1,4]oxazino)[2,3-*b*:3',2'-*i*]phenoxazin-4-ium,
wobei das jeweilige Gegenion frei wählbar ist.

In einigen Ausführungsformen der Erfindung kann es bevorzugt sein, wenn die Verbindungen der Formel (I) zumindest einen, vorzugsweise mehrere, wie bspw. zwei, drei, vier, fünf oder sechs ¹⁹F Substituenten aufweisen.

In einigen Ausführungsformen der Erfindung kann es bevorzugt sein, wenn die Verbindungen zumindest einen, vorzugsweise einen, zwei oder drei Substituenten aufweisen, die ausgewählt sind aus ⁷⁶Br, ⁷⁵Br oder ¹⁸F. In letzterem Fall kann es bevorzugt sein, wenn zwei oder drei Substituenten vorliegen, dass es sich um identische Substituenten handelt.

In einigen Ausführungsformen der Erfindung kann es vorteilhaft sein, wenn die Substituenten die ausgewählt sind aus ⁷⁶Br, ⁷⁵Br, ¹⁹F und ¹⁸F, an die durch R₁ und R₂ dargestellten Reste gebunden sind.

Gegenstand der Erfindung ist weiterhin ein Verfahren zur Herstellung der tracer wobei eine Verbindung der Formel 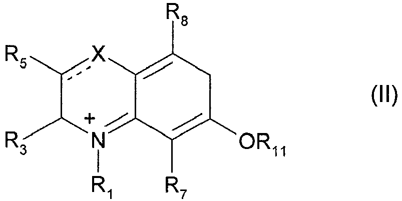 in welcher,
R₁, R₃, R₅, R₇ und R₈ die in Anspruch 1 definierte Bedeutung aufweisen, und
R₁₁ für Wasserstoff, (C₁-C₄)-Alkyl oder Phenylcarbonyl steht,
mit einer Verbindung der Formel 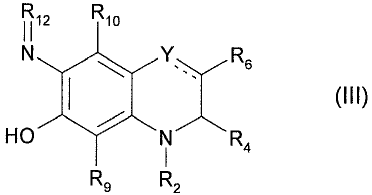 in welcher
R₂, R₄, R₆, R₉ und R₁₀ die in Anspruch 1 definierte Bedeutung aufweisen, und
R₁₂ für Oxo oder p-Nitrophenyl-N steht,
umgesetzt wird.

Die Umsetzung erfolgt im Allgemeinen in einem polaren Lösungsmittel in Gegenwart eine Säure, bevorzugt in einem Temperaturbereich von 60°C bis zum Rückfluss des Lösungsmittels bei Normaldruck.

Polare Lösungsmittel sind bspw. niedere Alkohole wie Methanol, Ethanol, n-Propanol oder Isopropanol, wobei Ethanol bevorzugt ist.

Säuren sind vorzugsweise Mineralsäuren wie bspw. Schwefelsäure, Salpetersäure, Chlorwasserstoffsäure oder Bromwasserstoffsäure, wobei Chlorwasserstoffsäure bevorzugt ist.

Die Verbindungen der Formel (III) können z.B. hergestellt werden, indem eine Verbindung der Formel (II), in der R₁₁ für Wasserstoff steht, mit 4-Nitro-phenyldiazonium-tetrafluoroborat umgesetzt wird.

Die Umsetzung erfolgt im Allgemeinen in einem polaren Lösungsmittel in Gegenwart eine Säure, bevorzugt bei Raumtemperatur bei Normaldruck.

Polare Lösungsmittel sind bspw. niedere Alkohole wie Methanol, Ethanol, n-Propanol oder Isopropanol, wobei Methanol bevorzugt ist.

Säuren sind vorzugsweise Mineralsäuren, insbesondere verdünnte Mineralsäuren wie bspw. Schwefelsäure, Salpetersäure, Chlorwasserstoffsäure oder Bromwasserstoffsäure, wobei verdünnte Schwefelsäure bevorzugt ist.

Die Verbindungen der Formel (II), in denen R₁₁ für Wasserstoff steht, können analog zu bekannten Verfahren (siehe z.B. K.-H. König, Chem. Berichte, 1959, 92 (2), 257-267 und A. Y. Berlin, J. Org. Chem. USSR, 1958, 2355-2359) aus kommerziell erhältlichen Verbindungen hergestellt werden.

Die Verbindungen der Formel (II), in denen R₁₁ für (C₁-C₄)-Alkyl oder Phenylcarbonyl steht, können unter Verwendung üblicher Schutzgruppen aus Verbindungen der Formel (II) hergestellt werden, in denen R₁₁ für Wasserstoff steht.

Die in den erfindungsgemäßen tracern vorhandenen ⁷⁶Br, ⁷⁵Br, ¹⁹F bzw. ¹⁸F Substituenten können bereits in den Ausgangsverbindungen vorliegen oder während der Synthese eingeführt werden.

Es kann hierbei im Rahmen der Erfindung vorteilhaft sein, in einer Zwischenstufe eine Verbindung der Formel (II) oder (III) zu synthetisieren, in der R₁ oder R₂ für ein Hydroxy-(C₁-C₂)-alkyl steht, in einer weiteren Stufe die Hydroxygruppe von R₁ oder R₂ in eine Abgangsgruppe umzuwandeln und diese dann zur Einführung eines ⁷⁶Br, ⁷⁵Br, ¹⁹F oder ¹⁸F Substituenten einer entsprechenden nukleophilen Substitutionsreaktion zu unterziehen.

Die Synthese der Verbindungen kann durch die nachfolgenden Syntheseschemata beispielhaft verdeutlicht werden.

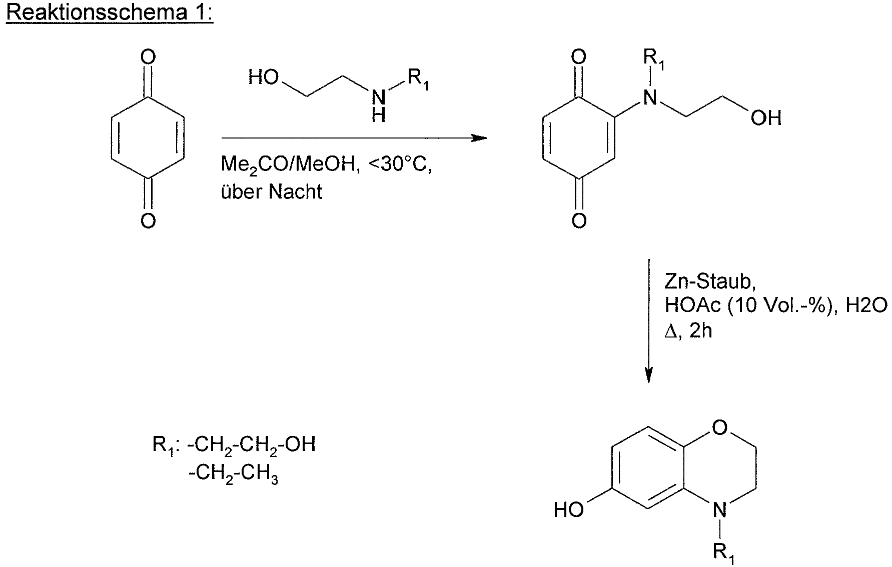

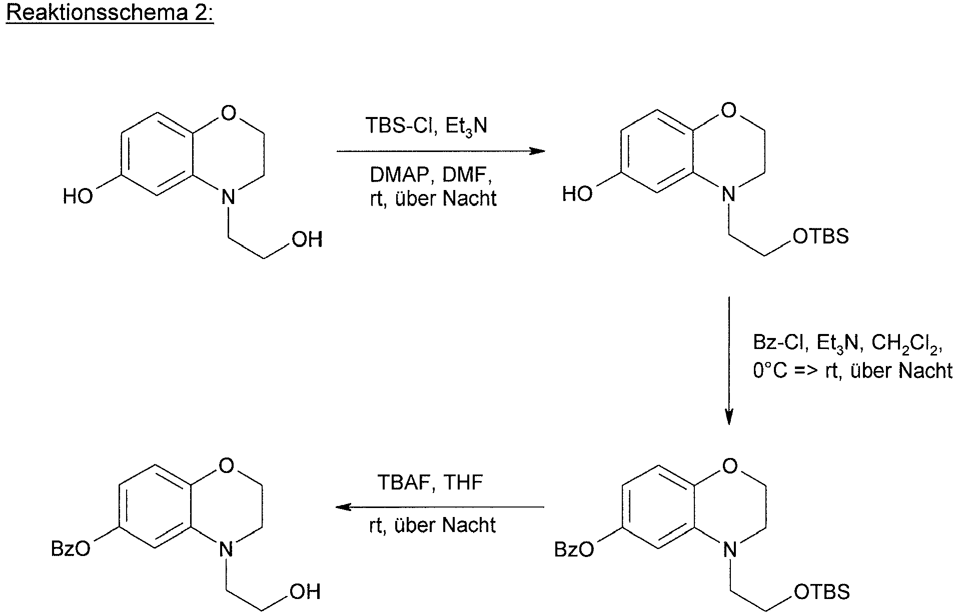

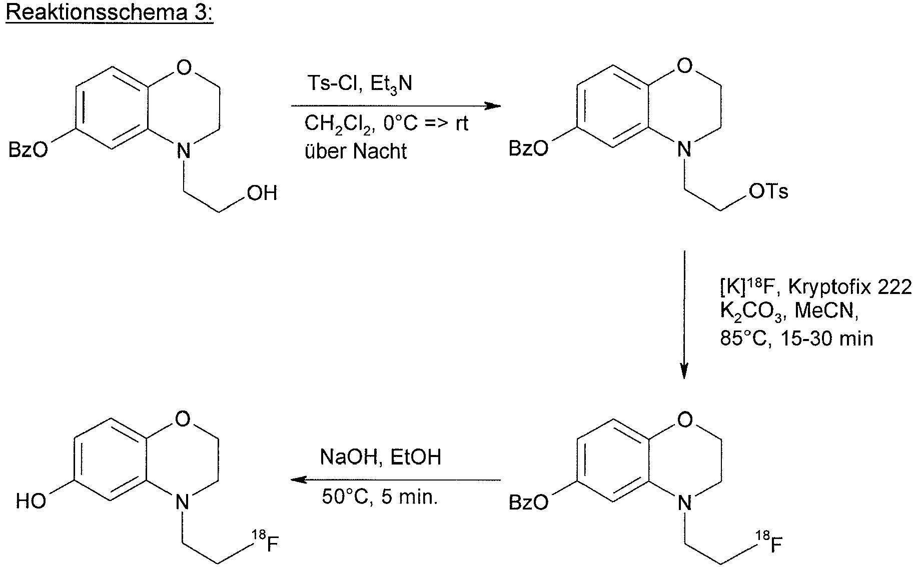

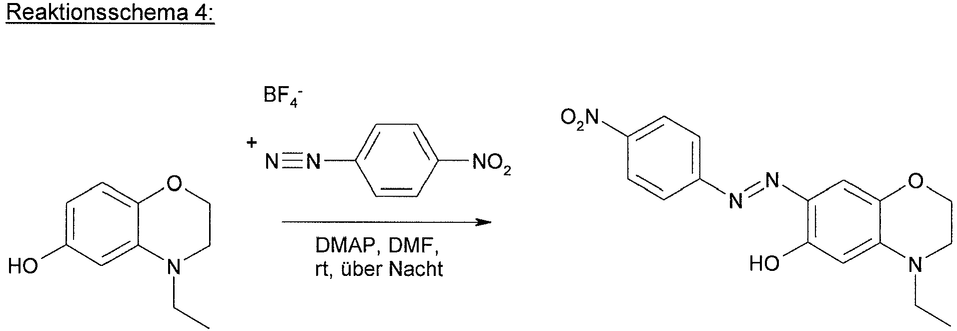

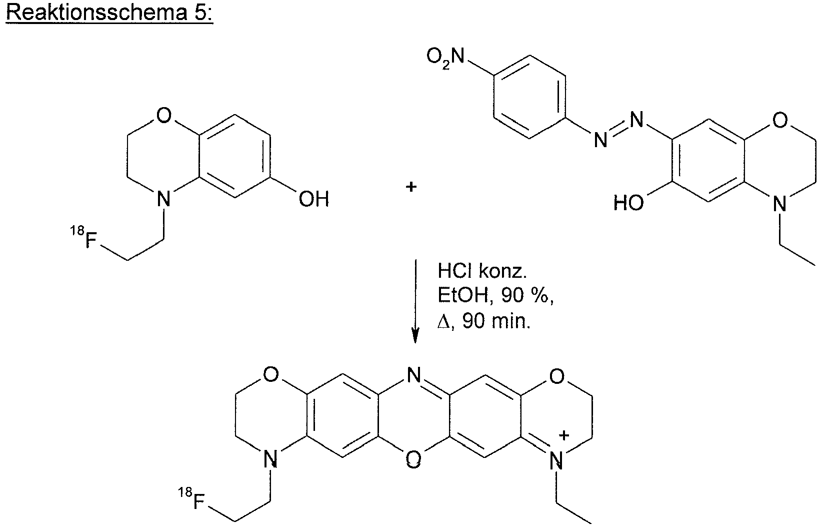

Die erfindungsgemäßen tracer zeigen ein nicht vorhersehbares, wertvolles pharmakologisches Wirkspektrum. Die erfindungsgemäßen tracer zeigen die Fähigkeit an Aß-Plaques zu binden, wodurch diese zur Detektion mittels MRT oder PET markiert werden.

Sie eignen sich daher zur Verwendung als Arzneimittel zur Diagnose von Krankheiten, vorzugsweise von Demenzerkrankungen und insbesondere der Alzheimer-Krankheit.

Weiterer Gegenstand der vorliegenden Erfindung ist daher die Verwendung der erfindungsgemäßen tracer in einem Verfahren zur Markierung von Aß-Plaques.

Weiterer Gegenstand der vorliegenden Erfindung ist die Verwendung der erfindungsgemäßen tracer in einem Verfahren zur Diagnose der Alzheimer-Krankheit.

Weiterer Gegenstand der vorliegenden Erfindung ist die Verwendung der erfindungsgemäßen tracer zur Herstellung eines Arzneimittels zur Diagnose von Krankheiten, vorzugsweise von Demenzerkrankungen und insbesondere der Alzheimer-Krankheit.

Weiterer Gegenstand der vorliegenden Erfindung ist ein Verfahren zur Diagnose von Krankheiten, vorzugsweise von Demenzerkrankungen und insbesondere der Alzheimer-Krankheit, das die Verabreichung eines tracers an einen Menschen oder ein Tier, der (das) dieses benötigt, aufweist.

Weiterer Gegenstand der vorliegenden Erfindung ist ein Verfahren zur Markierung von Aβ-Plaques, das das Inkontaktbringen eines tracers mit biologischem Material, insbesondere Hirnparenchym und Hirnvaskulatur, aufweist.

Das zuvor genannte erfindungsgemäße Verfahren kann dabei sowohl *in vivo* z.B. zur Diagnose der Alzheimer-Krankheit, wie auch *in vitro,* z.B. zum Screening nach neuen Medikamenten, erfolgen.

Die erfindungsgemäßen tracer können systemisch und/oder lokal appliziert werden. Bevorzugt können die erfindungsgemäßen Verbindungen parenteral verabreicht werden.

Für die gewünschten Applikationswege können die erfindungsgemäßen tracer in geeigneten Applikationsformen verabreicht werden.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Die erfindungsgemäßen tracer können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Weiterer Gegenstand der vorliegenden Erfindung sind Arzneimittel, die mindestens einen tracer üblicherweise zusammen mit einem oder mehreren inerten, nichttoxischen, pharmazeutisch geeigneten Hilfsstoffen enthalten, sowie deren Verwendung zu den zuvor genannten Zwecken.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachstehend in Bezug auf die folgenden nicht einschränkenden Beispiele sowie die Zeichnungen näher beschrieben und erläutert. Es zeigen:
- Fig. 1:: OI-Scans von transgenen Alzheimermäusen (rechts) und Kontrolltieren (links), Alter jeweils 12 Monate, nach Verabreichung von 0.1 mg/kg Körpergewicht der Verbindung von Beispiel 3, gemessen 120 min, nach der Injektion
- Fig. 2:: OI-Scans von transgenen Alzheimermäusen (rechts) und Kontrolltieren (links) nach Verabreichung von 0.1 mg/kg Körpergewicht der Verbindung von Beispiel 3, für Tiere im Alter von 2 Monaten (obere Reihe) und 12 Monaten (untere Reihe),
- Fig. 3:: ein Diagramm, in dem die in OI-Scans für die Anreicherung der Verbindung von Beispiel 3 gemessenen Signale gegen das Lebensalter transgener Alzheimermäuse aufgetragen sind, und
- Fig. 4:: ein Diagramm, in dem die in MRT-Scans für die Anreicherung der Verbindung von Beispiel 1 gemessenen Signale, sowie Signale entsprechender Messungen der Fluoreszenz gegen die Messdauer aufgetragen sind.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### Abkürzungsverzeichnis

- Aß-Plaques: Amyloid-beta-Peptid-Ablagerungen
- Bz-Cl: Benzoesäure-chlorid
- DMAP: 4-Dimethylamino-pyridin
- DMF: N,N-Dimethylformamid
- GC/MS: Gaschromatographie gekoppelt mit Massenspektrometrie
- Kryptofix222: 4,7,13,16,21,24-Hexaoxa-1, 10-diazabicyclo[8.8.8]-hexacosan
- MRT: Magnet Resonanz Tomographie
- MW: Molekulargewicht
- OI: optische Bildgebung
- PE: Petrolether bzw. Petroleumbenzin
- PET: Positronen Emissions Tomographie
- PIB: Pittsburgh Compound B
- TBAF: Tetrabutylammonium-fluorid-trihydrat
- TBS-CI: Chlor-*tert*-butyl-dimethylsilan
- THF: Tetrahydrofuran
- Ts-Cl bzw. Tosyl-chlorid: 4-Toluolsulfonsäure-chlorid

### Beispiele

### A. Analytische Geräte und Methoden:

### GC/MS

Agilent 7890 GC mit einem 5975C MSD mit EI, Säule: HP5-MS, Starttemperatur: 50°C, linearer Temperaturgradient auf 300°C innerhalb 20 min.

### Radio-HPLC

Agilent 1260 HPLC mit MWD-, RI- und Radiodetektor.

### LC/MS

Agilent 1200 HPLC mit MWD und MMI Single Quad MSD.

### DC-Scanner

Perkin Elmer Cyclone Plus Phosphor Imager.

### IR-S pektroskopie

Perkin Elmer FT-IR Spectrum ONE mit einer ATR Sampling Unit. Die Proben wurde entweder in fester oder flüssiger Form direkt mit der ATR Sampling Unit gemessen ohne die Verwendung von Presslingen oder Küvetten.

### UV/Vis-Spektroskopie

Perkin Elmer Lambda 25 UV/Vis Spektrometer. Die Spektren wurden in 3 mL Quarzglasküvetten aufgenommen mit Uvasol Methanol oder TraceSelect Wasser als Lösungsmittel.

### Fluoreszenzspektroskopie

Perkin Elmer LS 45 Fluoreszenz-Spektrometer. Die Spektren wurde in 3 mL Quarzglasküvetten aufgenommen mit Uvasol Methanol oder TraceSelect Wasser als Lösungsmittel.

### B. Ausgangsverbindungen und Intermediate:

### Beispiel 1A

### 2-(Ethyl(2-hydroxyethyl)amino)-1,4-benzochinon

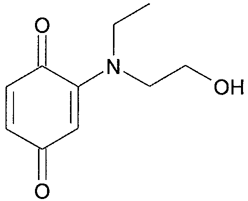

Zu einer Suspension von 1,4-Benzochinon (0.3 mol) in 72 ml Aceton wird bei Raumtemperatur eine Lösung von 2-(Ethylamino)ethanol (0.15 mol) in 18 mL Methanol so zugetropft, dass die Temperatur 30°C nicht übersteigt. Nach vollständiger Zugabe der methanolischen Lösung wird die dunkelbraune Suspension über Nacht bei Raumtemperatur gerührt. Danach wird die Suspension über einen Büchnertrichter abgesaugt und der erhaltene Rückstand mit wenig eiskaltem Methanol gewaschen. Die gelblichen bis braunen Kristalle werden anschließend im Exsikkator über Nacht getrocknet (Ausbeute: 16%).

MW: 181.19 g/mol

GC/MS: m/z = 181 (30.8%), 152 (8.5%), 151 (12.3%), 150 (100.0%), 122 (38.5%), 108 (7.3%), 94 (13.3%), 82 (9.2%), 81 (14.8%), 79 (8.1%), 68 (12.5%), 55 (7.7%), 54 (8.2%), 53 (19.9%), 52 (7.4%).

### Beispiel 2A

### 2-(Bis(2-hydroxyethyl)amino)-1,4-benzochinon

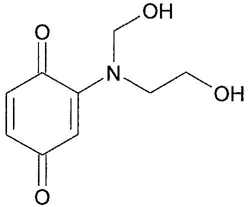

Die Synthese der Titelverbindung erfolgt analog zur Synthese der Verbindung aus Beispiel 1A, wobei Diethanolamin anstatt 2-(Ethylamino)ethanol verwendet wird. Man erhält die Titelverbindung in einer Ausbeute von 77%.

MW: 211.21 g/mol;

GC/MS: m/z = 211 (26.8%), 207 (19.6%), 195 (19.6%), 193 (15.0%), 182 (23.1%), 181 (13.7%), 180 (100.0%), 164 (37.7%), 162 (10.2%), 151 (13.4%), 150 (54.0%), 149 (12.7%), 136 (69.4%), 135 (10.1%), 122 (23.3%), 109 (16.(%), 108 (22.1%), 94 (10.(%), 82 (10.3%), 81 (16.7%), 80 (14.0%), 79 (11.2%), 68 (11.1%), 55 (17.6%), 54 (14.3%), 53 (24.5%), 52 (12.8%).

### Beispiel 3A

### 4-Methyl-3,4-dihydro-2H-benzo[b][1,4]oxazin-6-ol

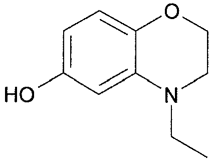

Zu einer Lösung der Verbindung aus Beispiel 1A (0.1 mol) in 250 mL 10%iger Essigsäure wird Zink-Staub (0.2 mol) gegeben und die Lösung für 30 min unter Rückfluss erhitzt, bis die DC-Analyse einen vollständigen Reaktionsumsatz zeigt. Die violette Suspension wird auf Raumtemperatur abkühlen gelassen, über einen Büchnertrichter abfiltriert und der Feststoff mit Wasser nachgewaschen. Das Filtrat wird mit konz. Ammoniak neutralisiert und dreimal mit 200 mL Ethylacetat extrahiert. Die vereinigten organischen Phasen werden einmal mit gesättigter NaCI-Lösung gewaschen, über Magnesiumsulfat getrocknet und am Rotationsverdampfer eingeengt, wobei ein dunkelbraunes Öl erhalten wird (Ausbeute: 53%).

MW: 165.19 g/mol

GC/MS: m/z = 166 (10.1%), 165 (100.0%), 164 (13.9%), 150 (53.7%), 136 (22.4%), 123 (5.7%), 109 (10.8%), 108 (5.3%), 82 (15.5%), 81 (12.8%), 80 (5.4%), 55 (7.4%), 53 (6.9%).

### Beispiel 4A

### 4-Ethyl-3,4-dihydro-2H-benzo[b][1,4]oxazin-6-ol

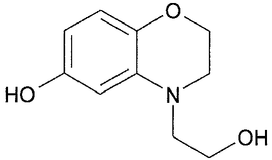

Die Synthese der Titelverbindung erfolgt analog zur Synthese der Verbindung aus Beispiel 3A, ausgehend von der Verbindung aus Beispiel 2A. Man erhält die Titelverbindung in einer Ausbeute von 83%.

MW: 179.22 g/mol

GC/MS: m/z = 180 (7.4%), 179 (63.1%), 165 (10.8%), 164 (100.0%), 150 (8.1%), 136 (22.5%), 123 (5.4%), 109 (7.7%), 108 (6.8%), 55 (5.1%).

### Beispiel 5A

### 4-(2-(tert-Butyldimethylsilyloxy)ethyl)-3,4-dihydro-2H-benzo[b]-[1,4]-oxazin-6-ol

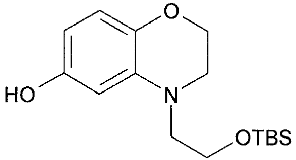

Zu einer Lösung der Verbindung aus Beispiel 4A (27.2 g, 0.139 mol, 1 equ.) in 300 mL DMF wird bei Raumtemperatur zunächst DMAP (∼30 mg, kat. Menge) und Et₃N (29 mL, 0.210 mol, 1.5 equ.) gegeben. Zu dieser Lösung wird dann portionsweise TBS-CI (22.03 g, 0.146 mol, 1.05 equ.) in fester Form langsam zugegen, wobei eine exotherme Reaktion zu beobachten ist. Die erhaltene Reaktionslösung wird bei Raumtemperatur für 48 Stunden gerührt. Zur Aufarbeitung wird die Reaktionslösung mit 1.5 L dest. Wasser versetzt und die erhaltene trübe Lösung viermal mit 250 mL EtOAc extrahiert. Die vereinigten organischen Phasen werden einmal mit 300 mL ges. NaCI-Lösung gewaschen, über MgSO₄ getrocknet und am Rotationsverdampfer eingeengt. Der erhaltene Rückstand wird säulenchromatographisch über Kieselgel mit PE/EtOAc/Et₃N = 750:250:10 als Laufmittel gereinigt, wobei ein dunkelbraunes Öl erhalten wird (Ausbeute: 94%).

MW: 309.48 g/mol

GC/MS: m/z = 310 (6.9%), 309 (29.8%), 252 (7.4%), 208 (7.9%), 165 (12.7%), 164 (100.0%), 136 (5.2%), 75 (4.0%), 73 (4.9%).

### Beispiel 6A

### 4-(2-(tert-Butyldimethylsilyloxy)ethyl)-3,4-dihydro-2H-benzo[b][1,4]oxazin-6-yl benzoat

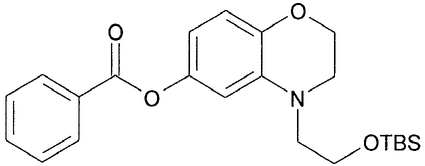

Zu einer Lösung der Verbindung aus Beispiel 5A (10.15 g, 32.8 mmol, 1 equ.) und Et₃N (4.8 mL, 34.5 mmol, 1.05 equ.) in 200 mL Dichlormethan wird bei 0°C eine 10%ige Lösung von Benzoylchlorid (4.0 mL, 34.5 mmol, 1.05 equ.) in Dichlormethan langsam über einen Tropftrichter zugegen. Nach beendeter Zugabe wird die Reaktionslösung im Eisbad belassen und unter langsamem Aufwärmen auf Raumtemperatur über Nacht gerührt. Zur Aufarbeitung wird die braune Reaktionslösung mit 400 mL dest. Wasser versetzt und für 5 min intensiv gerührt. Nach der Phasentrennung wird die wässrige Phase dreimal mit 200 mL Dichlormethan extrahiert. Die vereinigten organischen Phasen werden anschließend über MgSO₄ getrocknet und am Rotationsverdampfer eingeengt. Der Rückstand wird säulenchromatographisch über Kieselgel mit PE/EtOAc/Et₃N = 500:100:5 als Laufmittel gereinigt, wobei ein braunes Öl erhalten wird (Ausbeute: ∼quant.).

MW: 413.58 g/mol

GC/MS: m/z = 324 (5.3%), 323 (22.0%), 266 (4.2%), 222 (3.3%), 179 (12.0%), 178 (100.0%), 150 (4.6%), 135 (2.4%), 75 (2.6%), 73 (3.7%), 59 (2.1%).

### Beispiel 7A

### 4-(2-Hydroxyethyl)-3,4-dihydro-2H-benzo[b][1,4]oxazin-6-yl benzoat

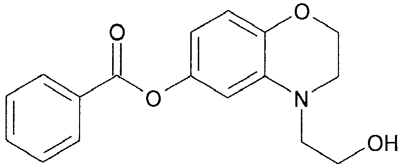

Zu einer Lösung der Verbindung aus Beispiel 6A (26.2 mmol, 1 equ.) in 100 mL THF wird bei 0°C eine Lösung von TBAF (27.5 mL, 27.5 mmol, 1.05 equ., 1M Lösung in THF) langsam zugegeben und die Reaktionslösung unter langsamem Erwärmen auf Raumtemperatur über Nacht gerührt. Zur Aufarbeitung wird die Reaktionslösung mit 150 mL dest. Wasser versetzt und für 5 min intensiv gerührt. Nach der Phasentrennung wird die wässrige Phase dreimal mit jeweils 100 mL Et₂O extrahiert. Die vereinigten organischen Phasen werden einmal mit 100 mL gesättigter NaCI-Lösung gewaschen, über MgSO₄ getrocknet und am Rotationsverdampfer eingeengt. Der Rückstand wird säulenchromatographisch über Kieselgel mit PE/EtOAc/Et₃N = 500:500:5 als Laufmittel gereinigt, wobei ein hellgelbes Öl erhalten wird (Ausbeute: 72%).

MW: 299.32 g/mol;

GC/MS: m/z = 300 (5.0%), 299 (26.8%), 270 (2.2%), 269 (18.0%), 268 (100.0%), 164 (2.9%), 108 (5.6%), 106 (5.1%), 105 (52.7%), 78 (2.2%), 77 (26.4%), 55 (2.3%), 51 (4.8%).

### Beispiel 8A

### 4-(2-Fluoroethyl)-3,4-dihydro-2H-benzo[b][1,4]oxazin-6-yl benzoat

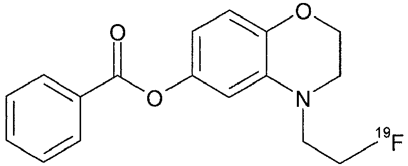

Zu einer Lösung der Verbindung aus Beispiel 7A (4.49 g, 15 mmol, 1 equ.) in 100 mL THF wird bei Raumtemperatur eine Lösung von Deoxofluor (13.0 mL, 30 mmol, 2.0 equ., 50%ige Lösung in THF) zugegeben und die Reaktionsmischung bei dieser Temperatur über Nacht gerührt. Zur Aufarbeitung wird die Reaktionsmischung mit ∼50g Kieselgel versetzt und am Rotationsverdampfer bis zur Trockne eingeengt. Der Rückstand wird säulenchromatographisch über Kieselgel mit PE/EtOAc/Et₃N = 300:150:5 als Laufmittel gereinigt, wobei ein gelbes Öl erhalten wird (Ausbeute: 79%)

MW: 301.31 g/mol

GC/MS: m/z = 302 (8.5%), 301 (44.6%), 269 (3.4%), 268 (18.5%), 168 (3.4%), 106 (8.4%), 105 (100.0%), 77 (28.9%), 51 (4.8%).

### Beispiel 9A

### 4-(2-Bromoethyl)-3,4-dihydro-2H-benzo[b][1,4]oxazin-6-yl-benzoat

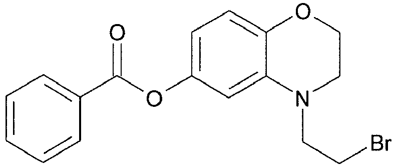

Zu einer Lösung der Verbindung von Beispiel 7A (1 g, 3.34 mmol, 1 equ.) in 30 mL CHCl₃ wird bei Raumtemperatur CBr₄ (2.23 g, 6.68 mmol, 2.0 equ.) gefolgt vom PPh₃ (1.75 g, 6.68 mmol, 2.0 equ.) zugegeben und die Reaktionsmischung bei dieser Temperatur für 60 min gerührt. Nachdem ein vollständiger Umsatz des Ausgangsmaterials mittels DC-Analyse festgestellt wird, wird die trübe Lösung filtriert und mit ∼20g Kieselgel versetzt. Die Suspension wird am Rotationsverdampfer bis zur Trockne eingeengt und der erhaltene Rückstand säulenchromatographisch über Kieselgel mit PE/EtOAc/Et₃N = 100:300:5 als Laufmittel gereinigt, wobei ein gelbes Öl erhalten wird (Ausbeute ∼quant.).

MW: 362.22 g/mol

GC/MS: m/z = 364 (4.3%), 363 (23.4%), 362 (4.7%), 361 (23.8%), 282 (2.6%), 269 (7.1%), 268 (39.9%), 109 (3.1%), 108 (3.5%), 107 (4.1%), 106 (8.4%), 105 (100.0%), 78 (2.3%), 77 (28.5%), 51 (4.7%).

### Beispiel 10A

### 4-(2-(Tosyloxy)ethyl)-3,4-dihydro-2H-benzo[b][1,4]oxazin-6-yl-benzoat

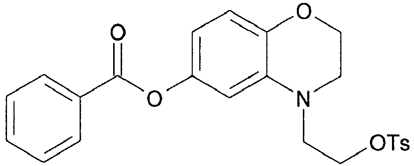

Zu einer Lösung der Verbindung aus Beispiel 7A (1 g, 3.34 mmol, 1 equ.) und Et₃N (0.7 mL, 5.0 mmol, 1.5 equ.) in 30 mL Dichlormethan wird bei 0°C eine Lösung von Tosylchlorid (0.7 g, 3.67 mmol, 1.1 equ.) in 10 mL Dichlormethan langsam zugetropft. Die erhaltene Reaktionslösung wird unter langsamem Erwärmen auf Raumtemperatur über Nacht gerührt. Zur Aufarbeitung wird die Reaktionslösung mit 100 mL Wasser versetzt und für 5 min intensiv gerührt. Nach der Phasentrennung wird die wässrige Phase dreimal mit 75 mL Dichlormethan extrahiert. Die vereinigten organischen Phasen werden einmal mit 100 mL gesättigter NaCI-Lösung gewaschen, über MgSO₄ getrocknet und am Rotationsverdampfer eingeengt, wobei ein braunes Öl erhalten wird. Der Rückstand wird säulenchromatographisch über Kieselgel mit PE/EtOAc/Et₃N = 300:100:5 als Laufmittel gereinigt, wobei ein hellbraunes Öl erhalten wird, welches sich beim Stehen im Kühlschrank verfestigt (Ausbeute: 74%).

MW: 453.51 g/mol

GC/MS: m/z = 342 (9.6%), 341 (35.7%), 281 (14.7), 269 (16.5%), 268 (100.0%), 208 (6.0%), 207 (25.6%), 148 (4.4%), 135 (4.6%), 108 (6.0%), 106 (8.8%), 105 (84.7%), 87 (8.4%), 78 (4.6%), 77 (27.1%), 73 (8.2%), 51 (4.8%).

### Beispiel 11A

### [F-18]-4-(2-Fluoroethyl)-3,4-dihydro-2H-benzo[b][1,4]oxazin-6-yl-benzoat

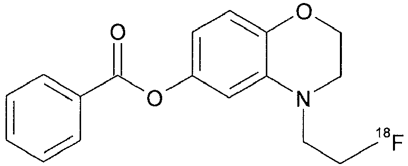

### 1. Schritt: Herstellung des radioaktiven Fluor-Reagenz

In einem 5 mL Reaktionsgefäß (reaction vial) werden 2 mL wässriges H¹⁸F vorgelegt und bei Raumtemperatur mit 100 µL einer 3.5%igen wässrigen Kaliumcarbonat-Lösung, 16 mg Kryptofix222 (Merck Darmstadt) und 1 mL Acetonitril (DNA Grade) versetzt. Die Lösung wird unter intensivem Rühren und einem Argon-Gasstrom auf 140°C erhitzt. In 4 min Intervallen werden dann viermal jeweils 1 mL Acetonitril zugegeben. 20 min nach Reaktionsstart wird kontrolliert, ob noch Flüssigkeit im Reaktionsgefäß vorhanden ist. Falls noch Flüssigkeit vorhanden ist wird die Reaktionsmischung weitere 4 Minuten gerührt. Anschließend lässt man das Reaktionsgefäß auf ∼30°C abkühlen und nimmt den Rückstand in 1 mL Acetonitril auf, wobei eine gelbe Lösung erhalten wird. Diese Lösung wird dann direkt für die Radiomarkierung im nächsten Schritt verwendet.

### 2. Schritt: Radiomarkierung

Zu einer Lösung der Verbindung von Beispiel 10A (4 mg) in 1 mL abs. Acetonitril wird die in Schritt 1 hergestellte Lösung von [K]¹⁸F zugegeben und die erhaltene Reaktionsmischung bei 85°C für 30 min gerührt. Nach Abkühlen auf ∼30°C wird die Reaktionsmischung auf eine präparative HPLC gegeben (Säule: Phenomenex Luna C18(2), 250x10 mm, Laufmittel: 60% Acetonitril / 40% Wasser, Flussrate: 7 mL/min, Detektor: UV und Radioaktivität) und das Produkt vom Ausgangsmaterial getrennt. Die Produktfraktion wird mit 70 mL dest. Wasser verdünnt und auf eine konditionierte Strata-X-SPE-Kartusche gegeben (Phenomenex, 10 mg Bettmaterial), wobei das radiomarkierte Produkt auf dem Bettmaterial zurückgehalten wird. Anschließend wird die Strata-X-Kartusche mit 5 mL Wasser gewaschen und überschüssiges Wasser mit 5 mL Luft entfernt. Zuletzt wird das Produkt mit 1 mL 96%igem Ethanol von der Kartusche eluiert (Ausbeute: 22%).

Die Identifikation der Titelverbindung erfolgte über Radio-DC mit PE/EtO₂ als Laufmittel.

### Beispiel 12A

### 4-(2-Fluoroethyl)-3,4-dihydro-2H-benzo[b][1,4]oxazin-6-ol

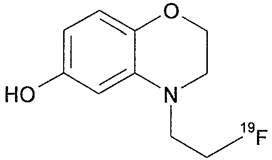

Zu einer Lösung der Verbindung aus Beispiel 8A (2 mmol, 1 equ.) in 30 mL abs. Methanol wird feste Natronlauge (MW: 40.0 g/mol, 84 mg, 2.1 mmol, 1.05 eq.) zugegeben und die Reaktionsmischung bei Raumtemperatur über Nacht gerührt. Zur Aufarbeitung wird die Reaktionslösung mit ~25g Kieselgel versetzt und am Rotationsverdampfer bis zur Trockne eingeengt. Der Rückstand wird säulenchromatographisch über Kieselgel gereinigt (Laufmittel: PE/EtOAc/Et₃N = 400:600:5), wobei ein Öl erhalten wird (Ausbeute: 99%).

MW: 197.21 g/mol;

GC/MS: m/z = 198 (10.4%), 197 (79.6%), 168 (4.9%), 165 (16.2%), 164 (100.0%), 136 (29.2%), 123 (7.4%), 109 (12.0%), 108 (10.4%), 94 (4.3%), 82 (6.7%), 81 (5.7%), 65 (4.4%), 55 (5.2%), 53 (5.6%);

### Beispiel 13A

### 4-(2-Bromoethyl)-3,4-dihydro-2H-benzo[b][1,4]oxazin-6-ol

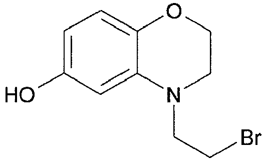

Die Synthese der Titelverbindung erfolgt analog zur Synthese der Verbindung aus Beispiel 12A, ausgehend von der Verbindung aus Beispiel 9A, wobei zur Aufreinigung PE/EtOAc/Et₃N = 700:300:25 als Laufmittel verwendet wird. Man erhält die Titelverbindung in einer Ausbeute von >90%.

Die Titelverbindung ist instabil und zersetzt sich innerhalb von 12 h bereits zu 50% bei -25°C. Die Titelverbindung wird daher direkt nach dem Isolieren weiter umgesetzt.

### MW: 258.11 g/mol;

GC/MS: m/z = 259 (20.7%), 257 (18.7%), 207 (4.4%), 178 (5.5%), 165 (11.7%), 164 (100.0%), 148 (3.9%), 136 (12.8%), 123 (4.4%), 109 (6.9%), 108 (4.6%), 94 (3.7%), 82 (4.3%), 55 (3.5%).

### Beispiel 14A

### 4-Ethyl-7-((4-nitrophenyl)diazenyl)-3,4-dihydro-2H-benzo[b][1,4]oxazin-6-ol

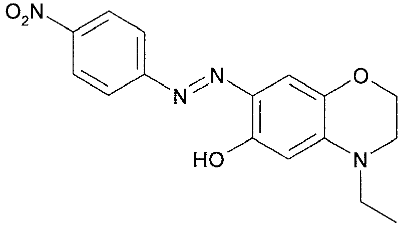

Zu einer Lösung der Verbindung aus Beispiel 3A (3 mmol, 1 equ.) in 10 mL Methanol wird bei Raumtemperatur eine Lösung von 4-Nitro-phenyl-diazoniumtetrafluoroborat (710 mg, 3 mmol, 1 equ.) in 2 mL 10%iger, wässriger Schwefelsäure zugegeben. Die tiefrote Reaktionslösung wird für 30 min bei Raumtemperatur gerührt und mit halbkonzentrierter Ammoniak-Lösung neutralisiert. Der erhaltene rote Niederschlag wird abfiltriert und mit kaltem dest. Wasser auf dem Filter gewaschen. Anschließend wird der rote Feststoff im Ölpumpen-Vakuum über Nacht getrocknet und aus n-Butanol umkristallisiert (Ausbeute: 82%).

MW: 328.32 g/mol;

LC/MS: m/z = 330 (19.3%), 329 (100.0%);

### Beispiel 15A

### 4-(2-Hydroxyethyl)-7-((4-nitrophenyl)diazenyl)-3,4-dihydro-2H-benzo[b][1,4]-oxazin-6-ol

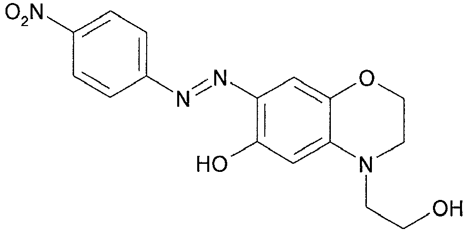

Die Synthese der Titelverbindung erfolgt analog zur Synthese der Verbindung aus Beispiel 14A, ausgehend von der Verbindung aus Beispiel 4A. Man erhält die Titelverbindung in einer Ausbeute von 94%.

MW: 344.32 g/mol;

LC/MS: m/z = 346 (19.0%), 345 (100.0%);

### C. Ausführungsbeispiele

### Beispiel 1

### 8-Ethyl-4-(2-fluoroethyl)-3,8,9,1 0-tetrahydro-2H-bis([1,4]oxazino)[2,3-b:3',2'-i]phenoxazin-4-ium Chlorid

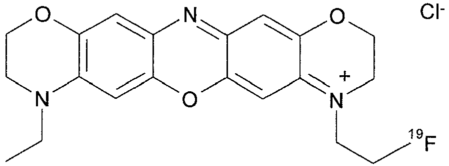

Zu einer Lösung der Verbindung aus Beispiel 14A (2 mmol, 1 equ.) und der Verbindung aus Beispiel 12A (3 mmol, 1.5 equ.) in 12 mL 90%igem Ethanol wird 1 mL 32%ige Salzsäure zugegeben und die Reaktionslösung für 60-240 min unter Rückfluss (∼80°C) erhitzt, bis die DC- bzw. LC/MS-Analyse einen vollständigen Reaktionsumsatz zeigt. Zur Aufarbeitung wird die Reaktionslösung mit konzentrierter Ammoniak-Lösung neutralisiert und am Rotationsverdampfer bis zur Trockne eingeengt. Der erhaltene Rückstand wird in Methanol aufgenommen, mit ∼10g Kieselgel versetzt, und erneut am Rotationsverdampfer bis zur Trockne eingeengt. Der grün-blaue Rückstand wird säulenchromatographisch über Kieselgel mit CH₂Cl₂/MeOH = 20:1 als Laufmittel gereinigt, wobei grünliche bis blaue Feststoffe erhalten werden (Ausbeute: 62%).

MW: 405.85 g/mol

LC/MS: m/z = 371 (22.9%), 370 (100.0%).

### Beispiel 2

### 4-(2-Bromoethyl)-8-ethyl-3,8,9,10-tetrahydro-2H-bis([1,4]oxazino)[2,3-b:3',2'-i]phenoxazin-4-ium Bromid

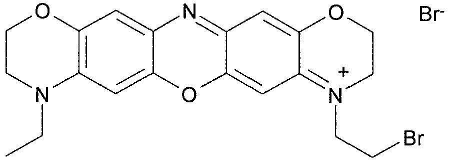

Die Synthese der Titelverbindung erfolgt analog zur Synthese der Verbindung aus Beispiel 1, ausgehend von den Verbindungen aus Beispiel 14A und Beispiel 13A. Nach Aufreinigung des Rohprodukts durch Säulenchromatographie an Kieselgel mit CH₂Cl₂/MeOH = 1000:50 als Laufmittel erhält man die Titelverbindung in einer Ausbeute von 24%.

Diese Verbindung fällt nicht unter den Schutzbereich von Anspruch 1 und soll lediglich als nicht-radioaktive Modellverbindung für ⁷⁶Br- und ⁷⁵Br-substituierte Verbindungen dienen.

MW: 511.12 g/mol

LC/MS: m/z = 436 (13.8%), 435 (46.4%), 434 (19.8%), 433 (57.0%), 247 (15.0%), 231 (10.1%), 230 (13.3%), 213 (13.7%), 207 (10.2%), 166 (100.0%), 138 (21.5%), 137 (65.1%), 125 (16.0%), 124 (49.5%);

### Beispiel 3

### [F-18]-8-Ethyl-4-(2-Fluoroethyl)-3,8,9,10-tetrahydro-2H-bis([1,4]-oxazino)[2,3-b:3',2'-i]phenoxazin-4-ium Chlorid

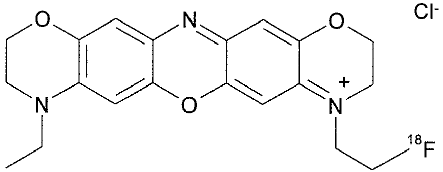

Zu einer ethanolischen Lösung der Verbindung aus Beispiel 11A werden bei Raumtemperatur 2 mg feste Natronlauge gegeben und die Reaktionsmischung bei 50°C für 5 min gerührt, wobei mittels Radio-DC ein vollständiger Umsatz beobachtet wird. Zu dieser Reaktionslösung wird eine Lösung bestehend aus der Verbindung aus Beispiel 14A (4 mg) und 50 µL konzentrierter Salzsäure gegeben und die Reaktionsmischung bei 75°C für 20 min gerührt. Die Reaktionsmischung wird per HPLC aufgereinigt und die Titelverbindung in einer Ausbeute von 8% erhalten.

Die Identifikation der Titelverbindung erfolgte über Radio-DC mit CH₂Cl₂/MeOH 10:1 als Laufmittel und Radio-HPLC mit der Verbindung aus Beispiel 1 als Vergleichssubstanz.

### Beispiel 4

### 4-(2-Fluoroethyl)-8-(2-hydroxyethyl)-3,8,9,10-tetrahydro-2H-bis([1,4]oxazino)[2,3-b:3',2'-i]phenoxazin-4-ium chlorid

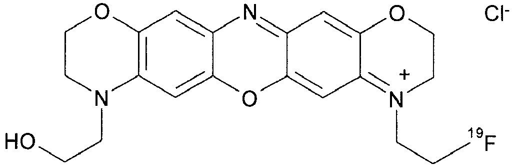

Die Synthese der Titelverbindung erfolgt analog zur Synthese der Verbindung aus Beispiel 1, ausgehend von den Verbindungen aus Beispiel 15A und Beispiel 12A. Nach Aufreinigung des Rohprodukts durch Säulenchromatographie an Kieselgel mit Gradient von CH₂Cl₂/MeOH = 20:1 auf CH₂Cl₂/MeOH = 1:1 erhält man die Titelverbindung in einer Ausbeute von 24%.

MW: 421.85 g/mol

LC/MS: m/z = 388 (7.6%), 387 (29.0%), 386 (100.0%), 385 (6.0%).

### D. Bewertung der physiologischen Aktivität

### Detektion von Aβ-Plaques mittels optischer Bildgebung (Ol) im Mausmodell

Zur Demonstration der selektiven Markierung von Aβ-Plaques durch radioaktiv markierte Verbindungen der Erfindung und die Sichtbarmachung der Markierung durch Ol wurden transgene APPPS1-Mäuse sowie nicht transgene Kontrollmäuse verschiedenen Alters (zur Verfügung gestellt von Prof. Mathias Jucker, Department of Cellular Neurology, Hertie-Institute for Clinical Brain Research, University of Tübingen) mit 0.1 mg/kg Körpergewicht der Verbindung von Beispiel 3 behandelt und dann mittels Ol untersucht. Die Verbindung wurde in physiologischer Kochsalzlösung (0.9 % NaCl) in einer Konzentration von 0.05 mg/ml formuliert und intravenös in die Mäuse injiziert. Nachfolgend wurden die Mäuse mittels Ol (Aequoria Hamamatsu Optical Imaging System) untersucht, das Signal wurde bei 650 nm detektiert.

Diese Versuche dienen dazu, die grundsätzliche Fähigkeit der erfindungsgemäßen tracer zu demonstrieren, an Aß-Plaques zu binden und ein zur Konzentration an Aß-Plaques korrelierendes Signal zu liefern. Es ist dem Fachmann dabei klar, dass diese Markierung dann in Abhängigkeit von der Art der Markierung durch jegliches Verfahren, sei es Ol, PET (z.B. bei ⁷⁶Br-, ⁷⁵Br- oder ¹⁸F-markierten Verbindungen) oder MRT (z.B. bei ¹⁹F-markierten Verbindungen) erfolgen kann.

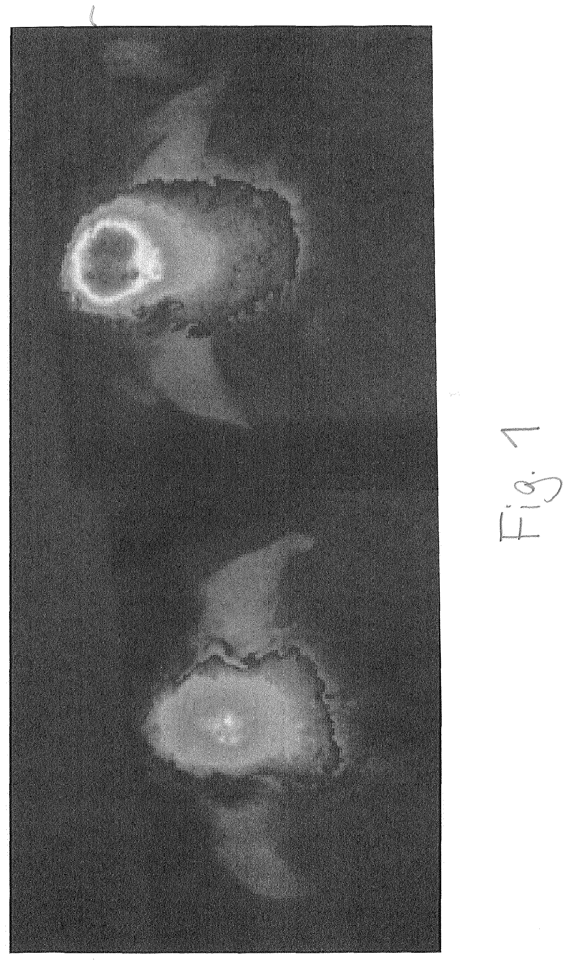

Bei diesen Versuchen hat sich gezeigt, dass sich die Verbindung von Beispiel 3 spezifisch in transgenen Mäusen anreichert während in Kontrollmäusen keine Anreicherung nachzuweisen war (siehe Fig. 1).

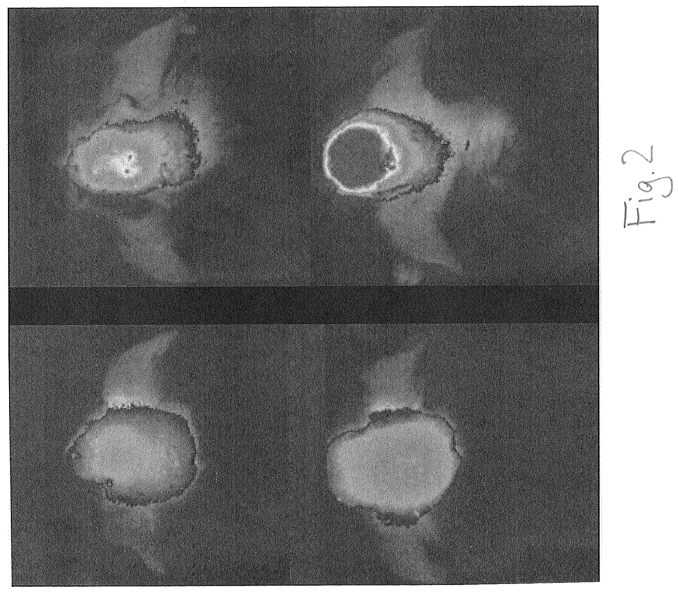

Es hat sich ferner gezeigt, dass die Anreicherung der Verbindung von Beispiel 3 mit dem Alter der transgenen Mäuse und somit der zunehmenden Bildung von Aß-Plaques zunimmt. Es zeigt sich dabei, dass in 2 Monate alten transgenen Mäusen - in denen es noch kaum zur Bildung von Aß-Plaques gekommen ist - keine Anreicherung nachzuweisen war, während im Alter von 12 Monaten eine deutliche Anreicherung sichtbar ist (siehe Fig. 2)

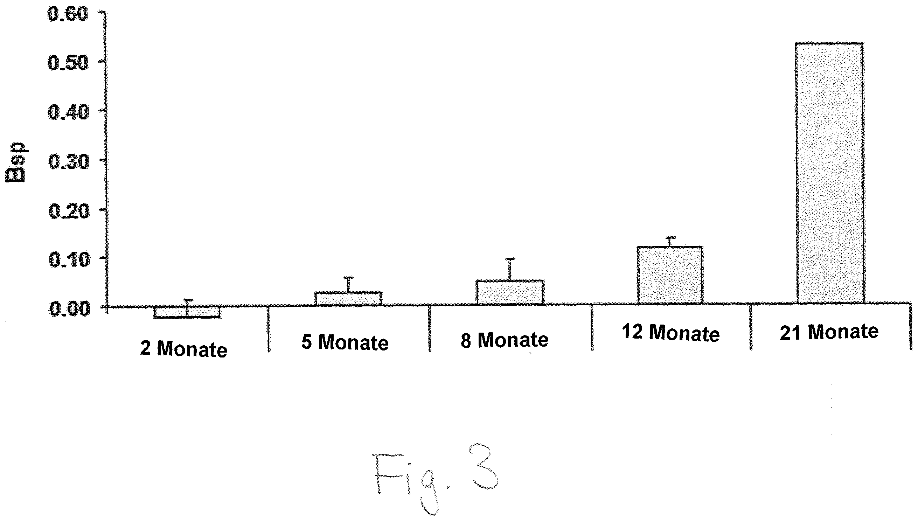

Die Signalstärke wurde als relatives Fluoreszenzsignal quantifiziert, wobei 30 min nach Injektion als Normierungszeitpunkt gewählt wurde. Hierbei wurde die Signalstärke nach Subtraktion der Autofluoreszenz prozentual berechnet (ergibt relative Intensitätswerte, Iᵣₑₗ) und anschließend der prozentuale Unterschied zwischen transgenen und Kontrollmäusen - im Folgenden als spezifische Bindung (Bₛₚ) bezeichnet - berechnet und über das Lebensalter der transgenen Mäuse aufgetragen (siehe Fig. 3). Es hat sich hierbei gezeigt, dass es eine deutliche Korrelation zwischen Lebensalter und somit Fortschreiten der Alzheimer-Krankheit und Signalstärke gibt, wobei insbesondere festgestellt wurde, dass es bei den erfindungsgemäßen tracern keine Sättigungs- oder Ceiling-Effekte dahingehend gibt, dass die Signalstärke ab einer gewissen Konzentration an Aβ-Plaques nicht mehr zunimmt. Ganz im Gegenteil wurde im Alter von 21 Monaten für die transgenen Mäuse ein besonders starkes Signal gemessen.

### Detektion von Aβ-Plaques mittels MRT im Mausmodell

Zur Demonstration der selektiven Markierung von Aβ-Plaques durch die mit Fluor markierten Verbindungen der Erfindung und die Sichtbarmachung der Markierung durch MRT wurden transgene APP23-Mäuse sowie nicht transgene Kontrollmäuse (zur Verfügung gestellt von Dr. Matthias Staufenbiel, Novartis Institutes for BioMedical Research, Basel, Schweiz) im Alter von 28 Monaten mit 2 mg/kg Körpergewicht der Verbindung von Beispiel 1 behandelt (die Verbindung wurde in physiologischer Kochsalzlösung (0.9% NaCl) in einer Konzentration von 1 mg/ml formuliert) und dann mittels MRT (ClinScan, Bruker BioSpin MRI, Ettlingen, Deutschland) sowie optischer Fluoreszenzmessungen untersucht (Aequoria Hamamatsu Optical Imaging System) und wie zuvor beschrieben ausgewertet. Für die MRT-Messungen wurde eine dreidimensionale Gradientenechosequenz (gre3D), die sowohl T1- als auch T2*-Anteile enthält, sowie eine quantitative T2-Wert Bestimmungssequenz (T2map) verwendet. Es konnte hierbei wiederum über die Messdauer von 90 Minuten eine spezifische Anreicherung in den transgenen Mäusen nachgewiesen werden. In Kontrollmäusen zeigte sich weder bei den MRT-Messungen noch bei den Fluoreszenzmessungen eine spezifische Anreicherung der Verbindung von Beispiel 1.

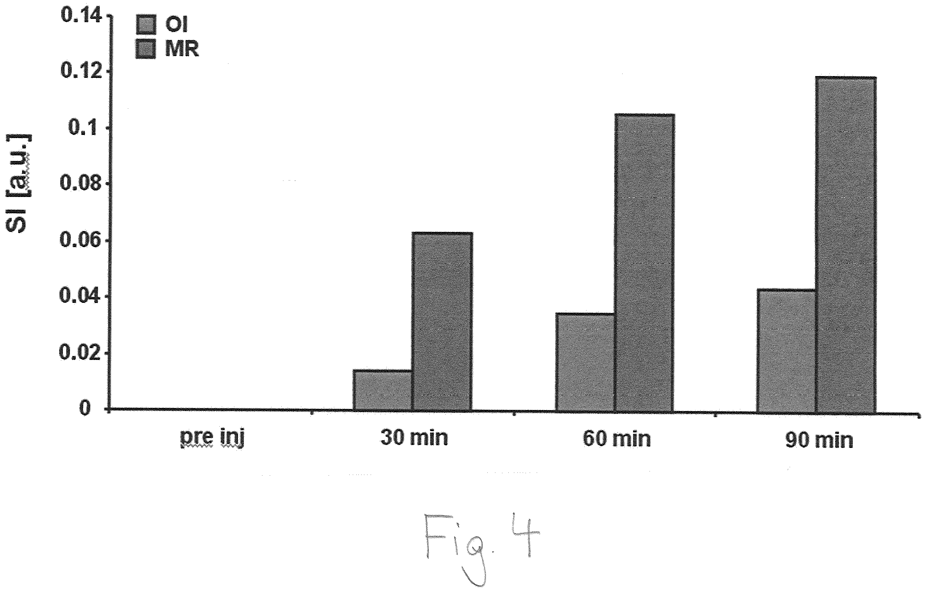

Die aus dem MRT erhaltenen Werte wurden mit den Werten aus der Fluoreszenzmessung verglichen, wobei sich eine signifikante Korrelation ergibt, wie diese auch aus dem Diagramm von Fig. 4 ersichtlich ist. Die *in vivo* gemachten Beobachtungen konnten auch nach einer Präparation der Gehirne *ex vivo* belegt werden.

### Zusammenfassung

Mit diesen Experimenten konnte die Eignung der erfindungsgemäßen tracer zur *in vivo* Markierung von Aβ-Plaques und deren nicht-invasive Detektion sowohl durch Ol als auch durch MRT demonstriert werden. Es hat sich hierbei gezeigt, dass die erfindungsgemäßen Verbindungen mit hoher Selektivität an Aβ-Plaques binden und über einen weiten Konzentrationsbereich zu einem zur Konzentration an Aβ-Plaques korrelierenden Signal führen. Aus den Ol-Untersuchungen unter Verwendung der Verbindung aus Beispiel 3 ergibt sich ,dass diese Verbindung selektiv an Aβ-Plaques bindet, wobei es einem Fachmann klar ist, dass sich diese Verbindung auf Grund des Vorliegens von 18F auch zur Untersuchung durch PET eignet und dort analoge Ergebnisse liefern wird. Die erfindungsgemäßen tracer sind somit zur nicht-invasiven Diagnose der Alzheimer-Krankheit und zur Verfolgung des Krankheitsverlaufs hervorragend geeignet.

## Patentansprüche

1. Radioaktiv markierter Tracer, aufweisend ein Salz der Verbindung der Formel 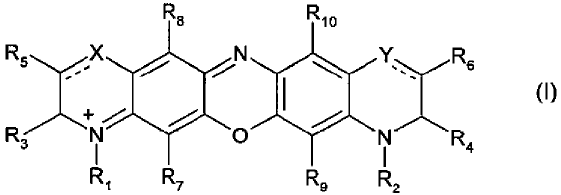 in welcher,
X und Y unabhängig voneinander für CH, CH₂, N, S oder O stehen,
wobei X und Y nicht gleichzeitig CH oder CH₂ sind,
wobei, wenn X für CH oder N steht, die gestrichelte Linie zwischen X und dem Nachbaratom eine Bindung darstellt, und
wobei, wenn Y für CH oder N steht, die gestrichelte Linie zwischen Y und dem Nachbaratom eine Bindung darstellt,
R₁ und R₂ unabhängig voneinander ausgewählt sind aus der Gruppe bestehend aus (C₁-C₂)-Alkyl, (C₁-C₂)-Alkoxy und (C₁-C₂)-Alkylsulfonyl,
wobei Alkyl, Alkoxy und Alkylsulfonyl mit ein bis drei Substituenten substituiert sein können, die ausgewählt sind aus der Gruppe bestehend aus ⁷⁶Br, ⁷⁵Br, ¹⁹F und ¹⁸F,
R₃, R₄, R₇, R₈, R₉ und R₁₀ unabhängig voneinander ausgewählt sind aus der Gruppe bestehend aus Wasserstoff, Halogen, Hydroxy, Amino, Cyano, Nitro, (C₁-C₄)-Alkyl, (C₁-C₄)-Alkylamino und (C₁-C₄)-Alkoxy,
wobei Alkyl und Alkoxy mit ein bis drei Substituenten substituiert sein können, die ausgewählt sind aus der Gruppe bestehend aus ⁷⁶Br, ⁷⁵Br, ¹⁹F und ¹⁸F,
R₅, wenn X für CH oder N steht, für einen Substituenten steht, der ausgewählt ist aus der Gruppe bestehend aus Wasserstoff, Halogen, Hydroxy, Amino, Cyano, Nitro, (C₁-C₄)-Alkyl, (C₁-C₄)-Alkylamino und (C₁-C₄)-Alkoxy,
wobei Alkyl und Alkoxy mit ein bis drei Substituenten substituiert sein können, die ausgewählt sind aus der Gruppe bestehend aus ⁷⁶Br, ⁷⁵Br, ¹⁹F und ¹⁸F,
R₅, wenn X für CH₂, S oder O steht, für zwei Substituenten steht, die unabhängig voneinander ausgewählt sind aus der Gruppe bestehend aus Wasserstoff, Halogen, Hydroxy, Amino, Cyano, Nitro, (C₁-C₄)-Alkyl, (C₁-C₄)-Alkylamino und (C₁-C₄)-Alkoxy,
wobei Alkyl und Alkoxy mit ein bis drei Substituenten substituiert sein können, die ausgewählt sind aus der Gruppe bestehend aus ⁷⁶Br, ⁷⁵Br, ¹⁹F und ¹⁸F,
R₆, wenn Y für CH oder N steht, für einen Substituenten steht, der ausgewählt ist aus der Gruppe bestehend aus Wasserstoff, Halogen, Hydroxy, Amino, Cyano, Nitro, (C₁-C₄)-Alkyl, (C₁-C₄)-Alkylamino und (C₁-C₄)-Alkoxy,
wobei Alkyl und Alkoxy mit ein bis drei Substituenten substituiert sein können, die ausgewählt sind aus der Gruppe bestehend aus ⁷⁶Br, ⁷⁵Br, ¹⁹F und ¹⁸F, und
R₆, wenn Y für CH₂, S oder O steht, für zwei Substituenten steht, die unabhängig voneinander ausgewählt sind aus der Gruppe bestehend aus Wasserstoff, Halogen, Hydroxy, Amino, Cyano, Nitro, (C₁-C₄)-Alkyl, (C₁-C₄)-Alkylamino und (C₁-C₄)-Alkoxy
wobei Alkyl und Alkoxy mit ein bis drei Substituenten substituiert sein können, die ausgewählt sind aus der Gruppe bestehend aus ⁷⁶Br, ⁷⁵Br, ¹⁹F und ¹⁸F,
wobei die Verbindung der Formel (I) zumindest einen Substituenten ausgewählt aus der Gruppe bestehend aus ⁷⁶Br, ⁷⁵Br, ¹⁹F und ¹⁸F aufweist,
oder Solvate des Salzes.

2. Radioaktiv markierter Tracer nach Anspruch 1, **dadurch gekennzeichnet, dass**
X und Y unabhängig voneinander für CH, CH₂, N, S oder O stehen,
wobei X und Y nicht gleichzeitig CH oder CH₂ sind,
wobei, wenn X für CH oder N steht, die gestrichelte Linie zwischen X und dem Nachbaratom eine Bindung darstellt, und
wobei, wenn Y für CH oder N steht, die gestrichelte Linie zwischen Y und dem Nachbaratom eine Bindung darstellt,
R₁ und R₂ unabhängig voneinander ausgewählt sind aus der Gruppe bestehend aus (C₁-C₂)-Alkyl, (C₁-C₂)-Alkoxy und (C₁-C₂)-Alkylsulfonyl,
wobei Alkyl, Alkoxy und Alkylsulfonyl mit ein bis drei Substituenten substituiert sein können, die ausgewählt sind aus der Gruppe bestehend aus ⁷⁶Br, ⁷⁵Br, ¹⁹F und ¹⁸F,
R₃, R₄, R₇, R₈, R₉ und R₁₀ unabhängig voneinander ausgewählt sind aus der Gruppe bestehend aus Wasserstoff, Halogen, Hydroxy, Amino, (C₁-C₂)-Alkyl, und (C₁-C₂)-Alkoxy,
wobei Alkyl und Alkoxy mit ein bis drei Substituenten substituiert sein können, die ausgewählt sind aus der Gruppe bestehend aus ⁷⁶Br, ⁷⁵Br, ¹⁹F und ¹⁸F,
R₅, wenn X für CH oder N steht, für einen Substituenten steht, der ausgewählt ist aus der Gruppe bestehend aus Wasserstoff, Halogen, Hydroxy, Amino, (C₁-C₂)-Alkyl, und (C₁-C₂)-Alkoxy,
wobei Alkyl und Alkoxy mit ein bis drei Substituenten substituiert sein können, die ausgewählt sind aus der Gruppe bestehend aus ⁷⁶Br, ⁷⁵Br, ¹⁹F und ¹⁸F,
R₅, wenn X für CH₂, S oder O steht, für zwei Substituenten steht, die unabhängig voneinander ausgewählt sind aus der Gruppe bestehend aus Wasserstoff, Halogen, Hydroxy, Amino, (C₁-C₂)-Alkyl, und (C₁-C₂)-Alkoxy,
wobei Alkyl und Alkoxy mit ein bis drei Substituenten substituiert sein können, die ausgewählt sind aus der Gruppe bestehend aus ⁷⁶Br, ⁷⁵Br, ¹⁹F und ¹⁸F,
R₆, wenn Y für CH oder N steht, für einen Substituenten steht, der ausgewählt ist aus der Gruppe bestehend aus Wasserstoff, Halogen, Hydroxy, Amino, (C₁-C₂)-Alkyl, und (C₁-C₂)-Alkoxy,
wobei Alkyl und Alkoxy mit ein bis drei Substituenten substituiert sein können, die ausgewählt sind aus der Gruppe bestehend aus ⁷⁶Br, ⁷⁵Br, ¹⁹F und ¹⁸F,
R₆, wenn Y für CH₂, S oder O steht, für zwei Substituenten steht, die unabhängig voneinander ausgewählt sind aus der Gruppe bestehend aus Wasserstoff, Halogen, Hydroxy, Amino, (C₁-C₂)-Alkyl, und (C₁-C₂)-Alkoxy,
wobei Alkyl und Alkoxy mit ein bis drei Substituenten substituiert sein können, die ausgewählt sind aus der Gruppe bestehend aus ⁷⁶Br, ⁷⁵Br, ¹⁹F und ¹⁸F,
wobei die Verbindung der Formel (I) zumindest einen Substituenten ausgewählt aus der Gruppe bestehend aus ⁷⁶Br, ⁷⁵Br, ¹⁹F und ¹⁸F aufweist.

3. Radioaktiv markierter Tracer nach Anspruch 1 oder 2, **dadurch gekennzeichnet, dass**
X und Y unabhängig voneinander für CH₂, S oder O stehen,
wobei X und Y nicht gleichzeitig CH₂ sind,
R₁ und R₂ unabhängig voneinander ausgewählt sind aus (C₁-C₂)-Alkyl, (C₁-C₂)-Alkoxy und (C₁-C₂)-Alkylsulfonyl,
wobei zumindest einer der durch R₁ und R₂ dargestellten Reste mit zumindest einem Substituenten substituiert ist, der ausgewählt ist aus der Gruppe bestehend aus ⁷⁶Br, ⁷⁵Br, ¹⁹F und ¹⁸F, und
R₃, R₄, R₅, R₆, R₇, R₈, R₉ und R₁₀ für Wasserstoff stehen.

4. Radioaktiv markierter Tracer nach einem der Ansprüche 1 bis 3, ausgewählt aus der Gruppe bestehend aus:
8-Ethyl-4-(2-fluoroethyl)-3,8,9,10-tetrahydro-2H-bis([1,4]-oxazino)[2,3-*b*:3',2'-*i*]phenoxazin-4-ium,
4-(2-Fluoroethyl)-8-(2-hydroxyethyl)-3,8,9,10-tetrahydro-2H-bis([1,4]oxazino)[2,3-*b*:3',2'-*i*]phenoxazin-4-ium,
[F-18]-8-Ethyl-4-(2-fluoroethyl)-3,8,9,10-tetrahydro-2H-bis([1,4]-oxazino)[2,3-*b*:3',2'-*i*]phenoxazin-4-ium,
[F-18]-4-(2-Fluoroethyl)-8-(2-hydroxyethyl)-3,8,9,10-tetrahydro-2H-bis([1,4]oxazino)[2,3-*b*:3',2'-*i*]phenoxazin-4-ium,
[Br-75]-8-Ethyl-4-(2-bromoethyl)-3,8,9,10-tetrahydro-2H-bis([1,4]-oxazino)[2,3-*b*:3',2'-*i*]phenoxazin-4-ium,
[Br-75]-4-(2-Bromoethyl)-8-(2-hydroxyethyl)-3,8,9,10-tetrahydro-2H-bis([1,4]oxazino)[2,3-*b*:3',2'-*i*]phenoxazin-4-ium,
[Br-76]-8-Ethyl-4-(2-bromoethyl)-3,8,9,10-tetrahydro-2H-bis([1,4]-oxazino)[2,3-*b*:3',2'-*i*]phenoxazin-4-ium, und
[Br-76]-4-(2-Bromoethyl)-8-(2-hydroxyethyl)-3,8,9,10-tetrahydro-2H-bis([1,4]oxazino)[2,3-*b*:3',2'-*i*]phenoxazin-4-ium.

5. Radioaktiv markierter Tracer, aufweisend eine Verbindung der Formel (I) oder Solvate des Salzes nach einem der Ansprüche 1 bis 4 zur Verwendung in einem Verfahren zur Markierung von Aß-Plaques.

6. Radioaktiv markierter Tracer, aufweisend eine Verbindung der Formel (I) oder Solvate des Salzes nach einem der Ansprüche 1 bis 4 zur Verwendung in einem Verfahren zur Diagnose der Alzheimer-Krankheit.

7. Verfahren zur Herstellung des radioaktiv markierten Tracers nach Anspruch 1, wobei eine Verbindung der Formel 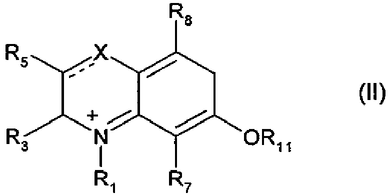 in welcher,
R₁, R₃, R₅, R₇ und R₈ die in Anspruch 1 definierte Bedeutung aufweisen, und
R₁₁ für Wasserstoff, (C₁-C₄)-Alkyl oder Phenylcarbonyl steht,
mit einer Verbindung der Formel 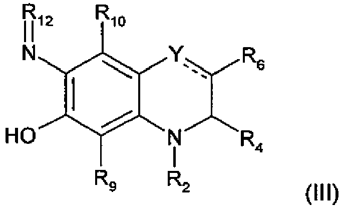 in welcher
R₂, R₄, R₆, R₉ und R₁₀ die in Anspruch 1 definierte Bedeutung aufweisen, und
R₁₂ für Oxo oder p-Nitrophenyl-N steht,
umgesetzt wird.

8. Verwendung des radioaktiv markierten Tracers nach einem der Ansprüche 1 bis 4 zur Herstellung eines Arzneimittels zur Diagnose von Krankheiten, vorzugsweise von Demenzerkrankungen und insbesondere der Alzheimer-Krankheit.

9. Arzneimittel, das mindestens den radioativ markierten Tracer nach einem der Ansprüche 1 bis 4 in Kombination mit mindestens einem inerten, nichttoxischen, pharmazeutisch geeigneten Hilfsstoff enthält.

10. Arzneimittel nach Anspruch 9 zur Verwendung in einem Verfahren zur Diagnose von Krankheiten, vorzugsweise von Demenzerkrankungen und insbesondere der Alzheimer-Krankheit.

11. Verfahren zur Diagnose von Krankheiten, vorzugsweise von Demenzerkrankungen und insbesondere der Alzheimer-Krankheit in Menschen und Tieren, das die Verabreichung des radioaktiv markierten Tracers nach einem der Ansprüche 1 bis 4 oder des Arzneimittels nach Anspruch 9 an einen Menschen oder ein Tier, der (das) dieses benötigt, aufweist.

12. Verfahren zur Markierung von Aß-Plaques, das das Inkontaktbringen des radioaktiv markierten Tracers nach einem der Ansprüche 1 bis 4 oder des Arzneimittels nach Anspruch 9 mit biologischem Material, insbesondere Hirnparenchym und Hirnvaskulatur, aufweist.

13. Verfahren nach Anspruch 12, **dadurch gekennzeichnet, dass** das Verfahren *in vitro* erfolgt.

14. Verfahren nach Anspruch 12, **dadurch gekennzeichnet, dass** das Verfahren *in vivo* erfolgt.

## Claims

1. Radioactively labeled tracer, comprising a salt of the compound of the formula 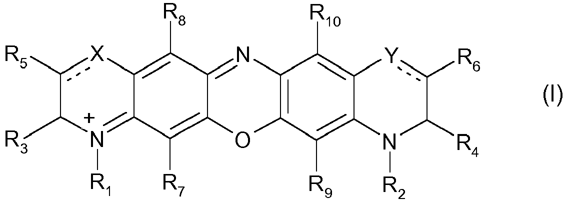 in which,
X and Y independently from one another represent CH, CH₂, N, S or O,
wherein X and Y are not simultaneously CH or CH₂,
wherein, if X represents CH or N, the dotted line between X and the neighboring atom represents a bond, and
wherein, if Y represents CH or N, the dotted line between Y and the neighboring atom represents a bond,
R₁ and R₂ independently from one another are selected from the group consisting of (C₁-C₂)-alkyl, (C₁-C₂)-alkoxy and (C₁-C₂)-alkyl sulfonyl,
wherein alkyl, alkoxy and alkyl sulfonyl can be substituted with one up to three substituents selected from the group consisting of ⁷⁶Br, ⁷⁵Br, ¹⁹F and ¹⁸F,
R₃, R₄, R₇, R₈, R₉ and R₁₀ independently from one another are selected from the group consisting of hydrogen, halogen, hydroxy, amino, cyano, nitro, (C₁-C₄)-alkyl, (C₁-C₄)-alkyl amino and (C₁-C₄)-alkoxy,
wherein alkyl and alkoxy can be substituted with one up to three substituents selected from group consisting of ⁷⁶Br, ⁷⁵Br, ¹⁹F and ¹⁸F,
R₅, if X represents CH or N, represents a substituent selected from the group consisting of hydrogen, halogen, hydroxy, amino, cyano, nitro, (C₁-C₄)-alkyl, (C₁-C₄)-alkyl amino and (C₁-C₄)-alkoxy,
wherein alkyl and alkoxy can be substituted with one up to three substituents selected from group consisting of ⁷⁶Br, ⁷⁵Br, ¹⁹F and ¹⁸F,
R₅, if X represents CH₂, S or O, represents two substituents independently from one another selected from the group consisting of hydrogen, halogen, hydroxy, amino, cyano, nitro, (C₁-C₄)-alkyl, (C₁-C₄)-alkyl amino and (C₁-C₄)-alkoxy,
wherein alkyl and alkoxy can be substituted with one up to three substituents selected from group consisting of ⁷⁶Br, ⁷⁵Br, ¹⁹F and ¹⁸F,
R₆, if X represents CH or N, represents a substituent selected from the group consisting of hydrogen, halogen, hydroxy, amino, cyano, nitro, (C₁-C₄)-alkyl, (C₁-C₄)-alkyl amino and (C₁-C₄)-alkoxy,
wherein alkyl and alkoxy can be substituted with one up to three substituents selected from group consisting of ⁷⁶Br, ⁷⁵Br, ¹⁹F and ¹⁸F, R₆, if Y represents CH₂, S or O, represents two substituents independently from one another selected from the group consisting of hydrogen, halogen, hydroxy, amino, cyano, nitro, (C₁-C₄)-alkyl, (C₁-C₄)-alkyl amino and (C₁-C₄)-alkoxy,
wherein alkyl and alkoxy can be substituted with one up to three substituents selected from group consisting of ⁷⁶Br, ⁷⁵Br, ¹⁹F and ¹⁸F,
wherein the compound of the formula (I) comprises at least one substituent selected from the group consisting of ⁷⁶Br, ⁷⁵Br, ¹⁹F und ¹⁸F,
or the solvates of the salt.

2. Radioactively labeled tracer of claim 1, **characterized in that**
X and Y independently from one another represent CH, CH₂, N, S or O,
wherein X and Y are not simultaneously CH or CH₂,
wherein, if X represents CH or N, the dotted line between X and the neighboring atom represents a bond, and
wherein, if Y represents CH or N, the dotted line between Y and the neighboring atom represents a bond,
R₁ and R₂ independently from one another are selected from the group consisting of (C₁-C₂)-alkyl, (C₁-C₂)-alkoxy and (C₁-C₂)-alkyl sulfonyl,
wherein alkyl, alkoxy and alkyl sulfonyl can be substituted with one up to three substituents selected from the group consisting of ⁷⁶Br, ⁷⁵Br, ¹⁹F und ¹⁸F,
R₃, R₄, R₇, R₈, R₉ and R₁₀ independently from one another are selected from the group consisting of hydrogen, halogen, hydroxy, amino, (C₁-C₂)-alkyl, and (C₁-C₂)-alkoxy,
wherein alkyl and alkoxy can be substituted with one up to three substituents selected from the group consisting of ⁷⁶Br, ⁷⁵Br, ¹⁹F und ¹⁸F,
R₅, if X represents CH or N, represents a substituent selected from the group consisting of hydrogen, halogen, hydroxy, amino, (C₁-C₂)-alkyl, and (C₁-C₂)-alkoxy,
wherein alkyl and alkoxy can be substituted with one up to three substituents selected from the group consisting of ⁷⁶Br, ⁷⁵Br, ¹⁹F and ¹⁸F,
R₅, if X represents CH₂, S or O, represents two substituents independently from one another selected from the group consisting of hydrogen, halogen, hydroxy, amino, (C₁-C₂)-alkyl, and (C₁-C₂)-alkoxy,
wherein alkyl and alkoxy can be substituted with one up to three substituents selected from the group consisting of ⁷⁶Br, ⁷⁵Br, ¹⁹F und ¹⁸F,
R₆, if Y represents CH or N, represents a substituent selected from the group consisting of hydrogen, halogen, hydroxy, amino, (C₁-C₂)-alkyl, and (C₁-C₂)-alkoxy,
wherein alkyl and alkoxy can be substituted with one up to three substituents selected from the group consisting of ⁷⁶Br, ⁷⁵Br, ¹⁹F und ¹⁸F,
R₆, if Y represents CH₂, S or O, represents two substituents independently from one another selected from the group consisting of hydrogen, halogen, hydroxy, amino, (C₁-C₂)-alkyl, and (C₁-C₂)-alkoxy,
wherein alkyl and alkoxy can be substituted with one up to three substituents selected from the group consisting of ⁷⁶Br, ⁷⁵Br, ¹⁹F und ¹⁸F,
wherein the compound of the formula (I) comprises at least a substituent selected from the group consisting of ⁷⁶Br, ⁷⁵Br, ¹⁹F and ¹⁸F.

3. Radioactively labeled tracer of claim 1 or 2, **characterized in that**
X and Y independently from one another represent CH₂, S or O,
wherein X and Y are not simultaneously CH₂,
R₁ and R₂ are independently from another selected from the group consisting of (C₁-C₂)-alkyl, (C₁-C₂)-alkoxy and (C₁-C₂)-alkyl sulfonyl,
wherein at least one of the residues represented by R₁ and R₂ is substituted with at least one substituent selected from the group consisting of ⁷⁶Br, ⁷⁵Br, ¹⁹F und ¹⁸F, and
R₃, R₄, R₅, R₆, R₇, R₈, R₉ and R₁₀ represent hydrogen.

4. Radioactively labeled tracer of any of claims 1 to 3, selected from the group consisting of:
8-Ethyl-4-(2-fluoroethyl)-3,8,9,10-tetrahydro-2H-bis([1,4]-oxazino)[2,3-*b*:3',2'-*i*]phenoxazine-4-ium,
4-(2-Fluoroethyl)-8-(2-hydroxyethyl)-3,8,9,10-tetrahydro-2H-bis([1,4]oxazino)[2,3-*b*:3',2'-*i*]phenoxazine-4-ium,
[F-18]-8-Ethyl-4-(2-fluoroethyl)-3,8,9,10-tetrahydro-2H-bis([1,4]-oxazino)[2,3-*b*:3',2'-*i*]phenoxazine-4-ium,
[F-18]-4-(2-Fluoroethyl)-8-(2-hydroxyethyl)-3,8,9,10-tetrahydro-2H-bis([1,4]oxazino)[2,3-*b*:3',2'-*i*]phenoxazine-4-ium,
[Br-75]-8-Ethyl-4-(2-bromoethyl)-3,8,9,10-tetrahydro-2H-bis([1,4]-oxazino)[2,3-*b*:3',2'-*i*]phenoxazine-4-ium,
[Br-75]-4-(2-Bromoethyl)-8-(2-hydroxyethyl)-3,8,9,10-tetrahydro-2H-bis([1,4]oxazino)[2,3-*b*:3',2'-*i*]phenoxazine-4-ium,
[Br-76]-8-Ethyl-4-(2-bromoethyl)-3,8,9,10-tetrahydro-2H-bis([1,4]-oxazino)[2,3-*b*:3',2'-*i*]phenoxazine-4-ium, and
[Br-76]-4-(2-Bromoethyl)-8-(2-hydroxyethyl)-3,8,9,10-tetrahydro-2H-bis([1,4]oxazino)[2,3-*b*:3',2'-*i*]phenoxazine-4-ium.

5. Radioactively labeled tracer, comprising a compound of the formula (I) or solvates of the salt of any of claims 1 to 4 for a use in a method for the labeling Aβ plaques.

6. Radioactively labeled tracer, comprising a compound of the formula (I) or solvates of the salt of any of claims 1 to 4 for a use in a method for diagnosing the Alzheimer's disease.

7. Method for the production of the radioactively labeled tracer of claim 1, wherein a compound of the formula 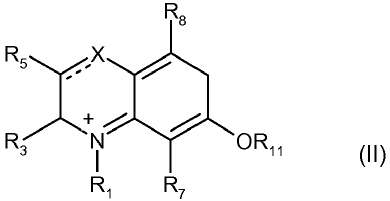 in which,
R₁, R₃, R₅, R₇ and R₈ have the meaning as defined in claim 1, and
R₁₁ is represented by hydrogen, (C₁-C₄)-alkyl or phenyl carbonyl,
is converted with a compound of the formula 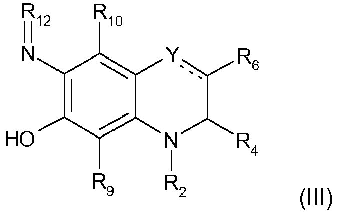 in which
R₂, R₄, R₆, R₉ and R₁₀ have the meaning as defined in claim 1, and
R₁₂ represents oxo or p-nitrophenyl-N.

8. Use of the radioactively labeled tracer of any of claims 1 to 4 for the production of a medicament for the diagnosis of diseases, preferably of dementia diseases and in particular of the Alzheimer's disease.

9. Medicament comprising at least the radioactively labeled tracer of any of claims 1 to 4 in combination with at least one inert non-toxic pharmaceutical acceptable excipient.

10. Medicament of claim 9 for a use in a method for the diagnosis of diseases, preferably of dementia diseases and in particular of the Alzheimer's disease.

11. Method for the diagnosis of diseases, preferably of dementia diseases and in particular the Alzheimer's disease in humans and animals, comprising the administration of the radioactively labeled tracer of any of claims 1 to 4 or the medicament of claim 9 to a human or animal in need thereof.

12. Method for the labeling of Aβ plaques comprising the contacting of the radioactively labeled tracer of any of claims 1 to 4 or the medicament of claim 9 with biological material, in particular brain parenchyma and brain vasculature.

13. Method of claim 12, **characterized in that** the method is realized *in vitro.*

14. Method of claim 12, **characterized in that** the method is realized *in vivo.*

## Revendications

1. Traceur marqué radioactivement, présentant un sel du composé de formule 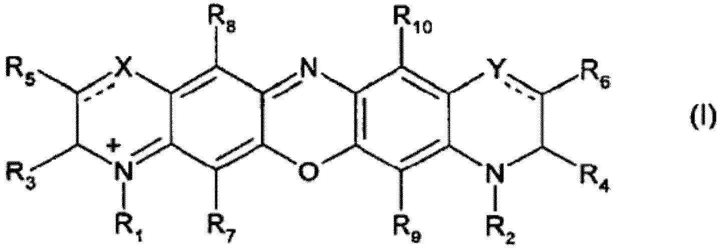 dans laquelle
X et Y désignent indépendamment l'un de l'autre CH, CH₂, N, S ou O,
dans lequel X et Y ne sont pas en même temps CH ou CH₂,
dans lequel, lorsque X désigne CH ou N, la ligne pointillée entre X et l'atome voisin représente une liaison, et
dans lequel, lorsque Y désigne CH ou N, la ligne pointillée entre Y et l'atome voisin représente une liaison,
R₁ et R₂ sont choisis indépendamment l'un de l'autre dans le groupe consistant en groupe alkyle (en C₁ à C₂), alcoxy (en C₁ à C₂) et alkylsulfonyle (en C₁ à C₂),
dans lequel les groupes alkyle, alcoxy et alkylsufonyle peuvent être substitués par un à trois substituants qui sont choisis dans le groupe consistant en ⁷⁶Br, ⁷⁵Br, ¹⁹F et ¹⁸F,
R₃, R₄, R₇, R₈, R₉ et R₁₀ sont choisis indépendamment les uns des autres dans le groupe consistant en atome d'hydrogène, d'halogène, en groupe hydroxy, amino, cyano, nitro, alkyle (en C₁ à C₄), alkylamino (en C₁ à C₄) et alcoxy (en C₁ à C₄),
dans lequel les groupes alkyle et alcoxy peuvent être substitués par un à trois substituants qui sont choisis dans le groupe consistant en ⁷⁶Br, ⁷⁵Br, ¹⁹F et ¹⁸F,
R₅, lorsque X désigne CH ou N, désigne un substituant qui est choisi dans le groupe consistant en atome d'hydrogène, d'halogène, en groupe hydroxy, amino, cyano, nitro, alkyle (en C₁ à C₄), alkylamino (en C₁ à C₄) et alcoxy (en C₁ à C₄),
dans lequel les groupes alkyle et alcoxy peuvent être substitués par un à trois substituants qui sont choisis dans le groupe consistant en ⁷⁶Br, ⁷⁵Br, ¹⁹F et ¹⁸F,
R₅, lorsque X désigne CH₂, S ou O, désigne deux substituants qui sont choisis indépendamment les uns des autres dans le groupe consistant en atome d'hydrogène, d'halogène, en groupe hydroxy, amino, cyano, nitro, alkyle (en C₁ à C₄), alkylamino (en C₁ à C₄) et alcoxy (en C₁ à C₄),
dans lequel les groupes alkyle et alcoxy peuvent être substitués par un à trois substituants qui sont choisis dans le groupe consistant en ⁷⁶Br, ⁷⁵Br, ¹⁹F et ¹⁸F,
R₆, lorsque Y désigne CH ou N, désigne un substituant qui est choisi dans le groupe consistant en atome d'hydrogène, d'halogène, en groupe hydroxy, amino, cyano, nitro, alkyle (en C₁ à C₄), alkylamino (en C₁ à C₄) et alcoxy (en C₁ à C₄),
dans lequel les groupes alkyle et alcoxy peuvent être substitués par un à trois substituants qui sont choisis dans le groupe consistant en ⁷⁶Br, ⁷⁵Br, ¹⁹F et ¹⁸F, et
R₆, lorsque Y désigne CH₂, S ou O, désigne deux substituants qui sont choisis indépendamment les uns des autres dans le groupe consistant en atome d'hydrogène, d'halogène, en groupe hydroxy, amino, cyano, nitro, alkyle (en C₁ à C₄), alkylamino (en C₁ à C₄) et alcoxy (en C₁ à C₄),
dans lequel les groupes alkyle et alcoxy peuvent être substitués par un à trois substituants qui sont choisis dans le groupe consistant en ⁷⁶Br, ⁷⁵Br, ¹⁹F et ¹⁸F,
dans lequel le composé de formule (I) présente au moins un substituant choisi dans le groupe consistant en ⁷⁶Br, ⁷⁵Br, ¹⁹F et ¹⁸F,
ou des solvates du sel.

2. Traceur marqué radioactivement selon la revendication 1, **caractérisé en ce que**
X et Y désignent indépendamment l'un de l'autre CH, CH₂, N, S ou O, dans lequel X et Y ne sont pas en même temps CH ou CH₂,
dans lequel, lorsque X désigne CH ou N, la ligne pointillée entre X et l'atome voisin représente une liaison, et
dans lequel, lorsque Y désigne CH ou N, la ligne pointillée entre Y et l'atome voisin représente une liaison,
R₁ et R₂ sont choisis indépendamment l'un de l'autre dans le groupe consistant en groupe alkyle (en C₁ à C₂), alcoxy (en C₁ à C₂) et alkylsulfonyle (en C₁ à C₂),
dans lequel les groupes alkyle, alcoxy et alkylsufonyle peuvent être substitués par un à trois substituants qui sont choisis dans le groupe consistant en ⁷⁶Br, ⁷⁵Br, ¹⁹F et ¹⁸F,
R₃, R₄, R₇, R₈, R₉ et R₁₀ sont choisis indépendamment les uns des autres dans le groupe consistant en atome d'hydrogène, d'halogène, en groupe hydroxy, amino, alkyle (en C₁ à C₂) et alcoxy (en C₁ à C₂),
dans lequel les groupes alkyle et alcoxy peuvent être substitués par un à trois substituants qui sont choisis dans le groupe consistant en ⁷⁶Br, ⁷⁵Br, ¹⁹F et ¹⁸F,
R₅, lorsque X désigne CH ou N, désigne un substituant qui est choisi dans le groupe consistant en atome d'hydrogène, d'halogène, en groupe hydroxy, amino, alkyle (en C₁ à C₂) et alcoxy (en C₁ à C₂),
dans lequel les groupes alkyle et alcoxy peuvent être substitués par un à trois substituants qui sont choisis dans le groupe consistant en ⁷⁶Br, ⁷⁵Br, ¹⁹F et ¹⁸F,
R₅, lorsque X désigne CH₂, S ou O, désigne deux substituants qui sont choisis indépendamment les uns des autres dans le groupe consistant en atome d'hydrogène, d'halogène, en groupe hydroxy, amino, alkyle (en C₁ à C₂) et alcoxy (en C₁ à C₂),
dans lequel les groupes alkyle et alcoxy peuvent être substitués par un à trois substituants qui sont choisis dans le groupe consistant en ⁷⁶Br, ⁷⁵Br, ¹⁹F et ¹⁸F,
R₆, lorsque Y désigne CH ou N, désigne un substituant qui est choisi dans le groupe consistant en atome d'hydrogène, d'halogène, en groupe hydroxy, amino, alkyle (en C₁ à C₂) et alcoxy (en C₁ à C₂),
dans lequel les groupes alkyle et alcoxy peuvent être substitués par un à trois substituants qui sont choisis dans le groupe consistant en ⁷⁶Br, ⁷⁵Br, ¹⁹F et ¹⁸F,
R₆, lorsque Y désigne CH₂, S ou O, désigne deux substituants qui sont choisis indépendamment les uns des autres dans le groupe consistant en atome d'hydrogène, d'halogène, en groupe hydroxy, amino, alkyle (en C₁ à C₂) et alcoxy (en C₁ à C₂),
dans lequel les groupes alkyle et alcoxy peuvent être substitués par un à trois substituants qui sont choisis dans le groupe consistant en ⁷⁶Br, ⁷⁵Br, ¹⁹F et ¹⁸F,
dans lequel le composé de formule (I) présente au moins un substituant choisi dans le groupe consistant en ⁷⁶Br, ⁷⁵Br, ¹⁹F et ¹⁸F.

3. Traceur marqué radioactivement selon la revendication 1 ou 2, **caractérisé en ce que**
X et Y désignent indépendamment l'un de l'autre CH₂, S ou O,
dans lequel X et Y ne sont pas en même temps CH₂,
R₁ et R₂ sont choisis indépendamment l'un de l'autre dans le groupe consistant en groupe alkyle (en C₁ à C₂), alcoxy (en C₁ à C₂) et alkylsulfonyle (en C₁ à C₂),
dans lequel au moins l'un des radicaux représentés par R₁ et R₂ est substitué par au moins un substituant qui est choisi dans le groupe consistant en ⁷⁶Br, ⁷⁵Br, ¹⁹F et ¹⁸F, et
R₃, R₄, R₅, R₆, R₇, R₈, R₉ et R₁₀ désignent un atome d'hydrogène.

4. Traceur marqué radioactivement selon l'une des revendications 1 à 3, choisi dans le groupe consistant en :
8-éthyl-4-(2-fluoroéthyl)-3,8,9,10-tétrahydro-2H-bis([1,4]-oxazino)[2,3-*b*:3',2'-*i*]phénoxazin-4-ium,
4-(2-fluoroéthyl)-8-(2-hydroxyéthyl)-3,8,9,10-tétrahydro-2H-bis([1,4]oxazino)[2,3-*b*:3',2'-*i*]phénoxazin-4-ium,
[F-18]-8-éthyl-4-(2-fluoroéthyl)-3,8,9,10-tétrahydro-2H-bis([1,4]-oxazino)[2,3-*b*:3',2'-*i*]phénoxazin-4-ium,
[F-18]-4-(2-fluoroéthyl)-8-(2-hydroxyéthyl)-3,8,9,10-tétrahydro-2H-bis([1,4]oxazino)[2,3-*b*:3',2'-*i*]phénoxazin-4-ium
[Br-75]-8-éthyl-4-(2-bromoéthyl)-3,8,9,10-tétrahydro-2H-bis([1,4]-oxazino)[2,3-*b*:3',2'-*i*]phénoxazin-4-ium,
[Br-75]-4-(2-bromoéthyl)-8-(2-hydroxyéthyl)-3,8,9,10-tétrahydro-2H-bis([1,4]oxazino)[2,3-*b*:3',2'-*i*]phénoxazin-4-ium,
[Br-76]-8-éthyl-4-(2-bromoéthyl)-3,8,9,10-tétrahydro-2H-bis([1,4]-oxazino)[2,3-*b*:3',2'-*i*]phénoxazin-4-ium, et
[Br-76]-4-(2-bromoéthyl)-8-(2-hydroxyéthyl)-3,8,9,10-tétrahydro-2H-bis([1,4]oxazino)[2,3-*b*:3',2'-*i*]phénoxazin-4-ium.

5. Traceur marqué radioactivement, présentant un composé de formule (I) ou des solvates du sel selon l'une des revendications 1 à 4, pour son utilisation dans un procédé de marquage de plaques Aβ.

6. Traceur marqué radioactivement présentant un composé de formule (I) ou des solvates du sel selon l'une des revendications 1 à 4, pour son utilisation dans un procédé de diagnostic de la maladie d'Alzheimer.

7. Procédé de production du traceur marqué radioactivement selon la revendication 1,
dans lequel on fait réagir un composé de formule 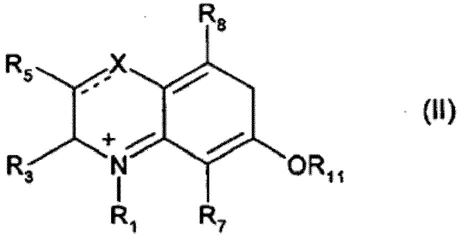 dans laquelle
R₁, R₃, R₅, R₇ et R₈ présentent la signification définie dans la revendication 1, et
R₁₁ désigne un atome d'hydrogène, un groupe alkyle (en C₁ à C₄) ou phénylcarbonyle,
avec un composé de formule 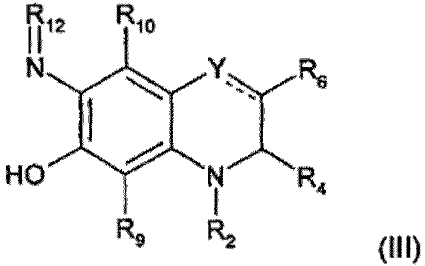
dans laquelle
R₂, R₄, R₆, R₉ et R₁₀ présentent la signification définie dans la revendication 1, et
R₁₂ désigne un groupe oxo ou p-nitrophényle-N.

8. Utilisation du traceur marqué radioactivement selon l'une des revendications 1 à 4, pour la production d'un médicament pour le diagnostic de maladies, de préférence de maladies de la démence et en particulier de la maladie d'Alzheimer.

9. Médicament qui contient au moins le traceur marqué radioactivement selon l'une des revendications 1 à 4, en combinaison avec au moins un adjuvant inerte, non toxique, pharmaceutiquement approprié.

10. Médicament selon la revendication 9, pour son utilisation dans un procédé de diagnostic de maladies, de préférence de maladies de la démence et en particulier de la maladie d'Alzheimer.

11. Procédé de diagnostic de maladies, de préférence de maladies de la démence et en particulier de la maladie d'Alzheimer chez l'être humain et l'animal, qui présente l'administration du traceur marqué radioactivement selon l'une des revendications 1 à 4 ou du médicament selon la revendication 9 à un être humain ou un animal qui en a besoin.

12. Procédé de marquage de plaques Aβ qui présente la mise en contact du traceur marqué radioactivement selon l'une des revendications 1 à 4 ou du médicament selon la revendication 9 avec un matériau biologique, en particulier le parenchyme cérébral et le système vasculaire cérébral.

13. Procédé selon la revendication 12, **caractérisé en ce que** le procédé s'effectue *in vitro.*

14. Procédé selon la revendication 12, **caractérisé en ce que** le procédé s'effectue *in vivo.*
